# EUROPEAN PATENT APPLICATION

(11) **EP 4 285 938 A1**
(43) Date of publication of application: **06.12.2023**
(21) Application number: 22746295.9
(22) Date of filing: 28.01.2022
(51) Int. Cl.: A61K 48/00, A61K 31/7105, A61K 38/15, A61K 31/138, A61K 45/06, A61P 35/00, A23L 33/13, A23L 33/18

(54) **PHARMACEUTICAL COMPOSITION FOR TREATING OR PREVENTING MALIGNANT BREAST CANCER**

(30) Priority: 29.01.2021 KR 20210012824; 27.01.2022 KR 20220012358
(71) Applicant: Korea Advanced Institute of Science and Technology, Daejeon 34141 (KR)
(72) Inventor: CHO, Kwang Hyun, Daejeon 34141 (KR); CHOI, Sea Rom, Daejeon 34141 (KR); HWANG, Chae Young, Daejeon 34141 (KR); LEE, Jong Hoon, Daejeon 34141 (KR)
(74) Representative: Cabinet Laurent & Charras
(86) International application number: PCT/KR2022/001622
(87) International publication number: WO 2022/164269

(57) **Abstract**

The present invention relates to a pharmaceutical composition for treating or preventing malignant breast cancer and, more particularly, to a composition for transdifferentiation of ER-negative breast cancer to luminal breast cancer, comprising an HDAC1/2 inhibitor and a BCL11A inhibitor as active ingredients, and use of the composition. According to the present invention, when target genes of the present invention are inhibited, BLT is induced so that basal-like or triplet-negative breast cancer is transdifferentiated to luminal A subtype breast cancer which is responsive to anticancer therapy, that is, being curable by hormone therapy. Accordingly, an effective and novel drug treatment that has not been conventionally attempted may be provided, thereby not only increasing the survival rate of patients through targeted therapy by realizing personalized medicine, but also contributing to an improvement in the quality of life that results from unnecessary anticancer drug therapy.

## Description

### [Technical Field]

The present invention relates to a pharmaceutical composition for treating or preventing malignant breast cancer.

### [Background Art]

Breast cancer is known to be cancer that is clinically and pathologically very complex and exhibits various aspects. Recently, as various targeted therapies that are effective in the treatment of breast cancer have been developed, breast cancer has also been gradually classified according to the presence or absence of the expression of a therapeutic target.

The overexpression of hormone receptors and human epidermal growth factor receptor 2 (HER2) are well-known targets of breast cancer treatment, and treatment of these targets has improved the prognosis of breast cancer. Recently, breast cancer is being evaluated at the genetic level according to gene expression aspects. Breast cancer was classified as Luminal A (estrogen receptor positive, HER2 negative); Luminal B (estrogen receptor positive, HER2 positive); HER2-enriched (estrogen receptor negative, HER2 positive); basal-like (estrogen receptor negative, HER2 negative) and normal type by DNA microarray method. In particular, it is known that basal-like breast cancer is mainly a group lacking hormone receptors and not expressing HER2/neu and has more early recurrence and poor prognosis compared to other groups. However, the classification method according to the gene expression pattern is difficult to apply clinically, and currently, a method of distinguishing breast cancer by the presence or absence of hormone receptor and HER2 expression through immunohistochemical staining is used.

Triple-negative breast cancer (TNBC) is breast cancer in which the expression of all human epidermal growth factor receptor 2 (HER2), estrogen receptor (ER), and progesterone receptor (PR) are negative in immunohistochemical staining, which accounts for 10 to 15% of all breast cancers. In particular, the rate of triple-negative breast cancer is high in Asian countries including South Korea. Triple-negative breast cancer does not strictly correspond to basal-like breast cancer, but since they present a very similar clinical course, they are considered identical in a real clinical setting. Triple-negative breast cancer has a relatively poor prognosis and low survival rate compared to non-triple-negative breast cancer patients.

The prognosis of breast cancer may vary depending on the molecular subtype of the breast tumor. For example, cancers in which estrogen receptor expression is positive (e.g., luminal type A and luminal type B) may have a better prognosis than breast cancers of other molecular subtypes.

In addition, cancers with a positive HER2 expression level (e.g., HER2 type, and luminal type B) may have a better prognosis than triple-negative cancers (e.g., basal type) according to the development of HER receptor inhibitors.

Furthermore, effective treatments may also differ depending on the molecular subtype of breast tumor. For example, tamoxifen, an estrogen receptor antagonist, can be an effective therapeutic agent for cancers with a positive estrogen receptor expression level (e.g., luminal type A, and luminal type B), and anti-HER2 antibodies and HER activating receptor tyrosine kinase inhibitors (e.g., Lapatinib) may be effective therapeutic agents for cancers with a positive HER2 expression level (e.g., HER2 type and luminal type B).

Previous studies have also attempted to develop effective treatments for triple-negative breast cancer patients, but have repeatedly failed due to the complex and dynamic nature of cancer. Therefore, even though diagnosis and treatment therapy for treating triple-negative breast cancer has been rapidly developed, the method of treating it still remains a difficult problem except for chemotherapy, so it is a state that requires multilateral tumor biologic identification.

Meanwhile, among these subtypes, luminal type A is the least aggressive breast cancer, and nearly 70% of breast cancer patients are diagnosed with luminal type A, and anti-ERα therapy can be applied by estrogen receptor alpha (ERα) expression.

Therefore, if it can transdifferentiate triple-negative breast cancer, which is refractory to conventional anticancer agents, into breast cancer subtypes that respond to conventional anticancer therapies, it can be an effective and new drug therapy, and this will contribute to realizing personalized medicine and improving the survival rate of patients and quality of life for patients suffering from unnecessary chemotherapy through targeted treatment.

### [Disclosure]

### [Technical Problem]

Under these circumstances, the present inventors have made intensive research efforts to develop an effective method for the treatment of triple-negative breast cancer (and basal-like breast cancer), which is a malignant breast cancer that exhibits ER-negative. As a result, the present inventors constructed a signal transduction network using epidemiological information and patient data and analyzed the growth signal flow pattern of disease-causing cells to derive HD AC 1/2 and BCL11A as effective drug targets for triple-negative breast cancer cells. Further, they discovered that when these HDAC1/2 and BCL11A were inhibited, the EGFR-ERK signaling pathway was inhibited, resulting in the expression of ERα, which was not seen in triple-negative breast cancer cells, as well as activation of related signaling pathways. Accordingly, when the triple-negative breast cancer cells in which HDAC1/2 and BCL11A were inhibited were treated with tamoxifen, a drug targeting ERα for patients with luminal A breast cancer, an excellent cancer cell killing effect was obtained, confirming high sensitivity to tamoxifen. This demonstrates that triple-negative breast cancer cells were reprogrammed or transdifferentiated into luminal A-type breast cancer that can be treated with conventionally known anticancer therapies, such as anti-ERα therapy, by the inhibition of HDAC1/2 and BCL11A of the present invention. Therefore, the present invention was completed by identifying that triple-negative breast cancer, which is refractory to conventional drugs, can respond (sensitize) to drugs by targeting a specific gene.

Accordingly, one object of the present invention is to provide a composition for transdifferentiating ER-negative breast cancer to luminal type breast cancer.

Further, another object of the present invention is to provide a composition for enhancing sensitivity to anticancer agent for ER-negative breast cancer.

Further, still another object of the present invention is to provide an anticancer adjuvant for treating or preventing ER-negative breast cancer.

Further, still another object of the present invention is to provide a pharmaceutical composition for treating or preventing ER-negative breast cancer.

Further, still another object of the present invention is to provide a food composition for preventing or alleviating ER-negative breast cancer.

Further, still another object of the present invention is to provide a method for inducing *in vitro* transdifferentiation of estrogen receptor-negative breast cancer to luminal type breast cancer.

Further, still another object of the present invention is to provide a method for screening a drug for enhancing sensitivity to anticancer agent for ER-negative breast cancer.

Further, still another object of the present invention is to provide a method for transdifferentiating ER-negative breast cancer to luminal type breast cancer.

Further, still another object of the present invention is to provide a method for treating or preventing ER-negative breast cancer.

### [Technical Solution]

The terms used in this specification are used only for the purpose of description and should not be construed as limiting. Singular expressions include plural expressions unless the context clearly dictates otherwise. It should be understood that as used herein, terms such as "comprise" or "have" are intended to designate that there is a feature, number, step, operation, component, part, or combination thereof described in the specification, but do not preclude the existence or addition of one or more other features, numbers, steps, operations, components, parts, or combinations thereof.

Unless defined otherwise, all terms used herein, including technical or scientific terms, have the same meaning as commonly understood by one of ordinary skill in the art to which these embodiments belong. Terms such as those defined in commonly used dictionaries should be interpreted as having a meaning consistent with the meaning in the context of the related art, and unless explicitly defined in this application, it is not to be construed in an ideal or overly formal sense.

Hereinafter, the present invention is described in detail.

According to one aspect of the present invention, the present invention provides a composition for transdifferentiating ER-negative breast cancer to luminal breast cancer, the composition including B-cell lymphoma/leukemia 11A (BCL11A) inhibitor and Histone deacetylase 1/2 (HDAC1/2) inhibitor as active ingredients.

In the present invention, the luminal type breast cancer may include at least one type selected from the group consisting of luminal A and luminal B.

As used herein, the term "breast cancer" means a mass made up of cancerous cells in the breast. Breast cancer can be a type of cancer originating in the mammary lobules that supply milk to the mammary gland or in the inner lining of the mammary gland. Cancers originating from mammary glands may be ductal carcinomas, and cancers originating from lobules may be lobular carcinomas. Occasionally, sites of metastasis by the breast cancer may include bone, liver, lungs, and brain. Breast cancer occurs in humans and other mammals. In humans, breast cancer most often occurs in women, but it can also occur in men. Treatment of breast cancer may include surgery, drug therapy (hormonal therapy and chemotherapy), radiation therapy, and/or immunotherapy/targeted therapy.

According to a preferred embodiment of the present invention, the ER-negative breast cancer is negative for at least one selected from the group consisting of progesterone receptor (PR) and human epidermal growth factor receptor 2 (HER2), and includes triple-negative breast cancer (TNBC) or basal-like breast cancer.

Specifically, breast cancer in the present invention may be classified as subtypes of luminal type A (estrogen receptor positive, HER2 negative); luminal type B (estrogen receptor positive, HER2 positive); HER2-enriched (estrogen receptor negative, HER2 positive); basal-like (estrogen receptor negative, HER2 negative); and triple-negative breast cancer (TNBC) (estrogen receptor negative, progesterone receptor negative, HER2 negative) according to the presence or absence of the expression of the target (receptor),

Of these, both basal-like breast cancer and triple-negative breast cancer are subtypes that lack hormone (estrogen) receptors and do not express HER2, and they are malignant breast cancer known to have more early recurrence and poor prognosis than the other subtypes, and it is determined that they are the same in clinical practice because they show similar clinical course.

Therefore, within the embodiment of the present invention, the basal-like or triple-negative described while referring to ER-negative breast cancer, which is the target, are regarded as including each other as long as the target gene of the present invention can achieve the desired effect, even if there is no special mention in the present invention.

The BCL11A inhibitor and HDAC1/2 inhibitor of the present invention may include any inhibitor as long as it can inhibit BCL11A and HDAC1/2, which are targets of the present invention, and for example, the inhibitors may include, but are not limited to, those that inhibit the expression of nucleotides of target genes BCL11A and HDAC1/2 or the activity of BCL11A and HDAC1/2 proteins.

According to a preferred embodiment of the present invention, the BCL11A inhibitor may include at least one selected from the group consisting of an antisense oligonucleotide, small interference RNA (siRNA), short hairpin RNA (shRNA), microRNA (miRNA) and ribozyme which complementarily bind to an mRNA of BCL11A gene, and the HDAC1/2 inhibitor may include at least one selected from the group consisting of an antisense oligonucleotide, small interference RNA (siRNA), short hairpin RNA (shRNA), microRNA (miRNA) and ribozyme which complementarily bind to an mRNA of HD AC 1/2 gene.

Further, the BCL11A inhibitor may include at least one selected from the group consisting of a compound, peptide, peptidomimetic, substrate analog, aptamer, and antibody which specifically bind to BCL11A protein, and the HDAC1/2 inhibitor may include at least one selected from the group consisting of a compound, peptide, peptidomimetic, substrate analog, aptamer, and antibody which specifically bind to HD AC 1/2 protein.

Further, the BCL11A inhibitor and the HDAC1/2 inhibitor of the present invention are characterized by reducing the expression level or activity of epidermal growth factor receptor (EGFR) and extracellular signal-regulated kinase 1/2 (ERK1/2) of ER-negative breast cancer, for example, triple-negative breast cancer (TNBC) or basal-like breast cancer (BLBC) and increasing the expression level or activity of estrogen receptor alpha (ERα).

According to one embodiment of the present invention, the present inventors constructed a molecular regulatory network model that can represent the biological conditions of basal-like breast cancer cells and luminal type A breast cancer cells, and applied a complex network control strategy to identify potential targets that could induce the basal-to-luminal transition (BLT) from the most invasive basal-like breast cancer type to the luminal type A. Further, the basic mechanism of BLT was explained by analyzing the logical relationship between major molecules in the network model. The present inventors discovered BCL11A and HDAC1/2 as new targets capable of reprogramming BLT and verified them through simulation analysis and cell line experiments.

In an embodiment, the present inventors constructed a signal transduction network using epidemiological information and respective patient data and analyzed the growth signal flow of disease-causing cells to derive HDAC1/2 and BCL11A as effective drug targets for triple-negative breast cancer cells. Further, they discovered that when these HDAC1/2 and BCL11A were inhibited, the EGFR-ERK signaling pathway was inhibited, resulting in the expression of ERα, which was not seen in triple-negative breast cancer cells, as well as activation of related signaling pathways. Accordingly, when the triple-negative breast cancer cells in which HD AC 1/2 and BCL11A were inhibited were treated with tamoxifen, a drug targeting ERα for patients with luminal A breast cancer, an excellent cancer cell killing effect was obtained, confirming high sensitivity to tamoxifen. This demonstrates that triple-negative breast cancer cells were reprogrammed or transdifferentiated into luminal type A breast cancer that can be treated with conventionally known anticancer therapies, such as anti-ERα therapy, by the inhibition of HDAC1/2 and BCL11A of the present invention. Therefore, it was identified that the sensitivity (sensitization) of triple-negative breast cancer, which is refractory to conventional drugs, can be enhanced to become sensitive to drugs (sensitization) by targeting a specific gene.

Accordingly, the present invention provides a method for inducing *in vitro* transdifferentiation of ER-negative breast cancer to luminal type breast cancer, the method including treating a BCL11A inhibitor and an HDAC1/2 inhibitor to an ER-negative breast cancer cell and a composition for enhancing sensitivity to an anticancer agent for breast cancer, the composition including the above-described composition for transdifferentiation of the present invention as an active ingredient.

Further, according to another aspect of the present invention, the present invention may provide an anticancer adjuvant for treating ER-negative breast cancer, the adjuvant including the BCL11A inhibitor and the HD AC 1/2 inhibitor as active ingredients.

In the present invention, the anticancer adjuvant means a substance that can enhance the anticancer effect of an anticancer agent by administering it in combination with an anticancer agent when administering an anticancer agent.

According to the present invention, since inhibition of HD AC 1/2 and BCL11A transdifferentiates triple-negative breast cancer cells into luminal A breast cancer cells that can be treated with anti-ERα therapy, the sensitivity of triple-negative breast cancer, which is conventionally anticancer agent-refractory, to anticancer agent, is increased to double the effect of anticancer agent.

The adjuvant of the present invention may be administered simultaneously, separately or sequentially with the anticancer agent. The order of administration of the anticancer adjuvant according to the present invention, that is, which of the anticancer agent and the anticancer adjuvant is to be administered simultaneously, separately or sequentially, can be determined by a doctor or an expert. This order of administration can vary depending on many factors.

According to another aspect of the present invention, the present invention provides a pharmaceutical composition for treating or preventing ER-negative breast cancer, the composition including a composition for transdifferentiating ER-negative breast cancer to luminal type breast cancer including the BCL11A inhibitor and the HDAC1/2 inhibitor as active ingredients; and an anticancer agent as active ingredients.

The anticancer agent includes all anticancer agents that can be used clinically, pharmaceutically, and biomedically, as long as the HDAC1/2 inhibitor and BCL11A inhibitor of the present invention can achieve the desired effect, but preferably, a hormone therapy agent for patients with luminal type A breast cancer, which is a hormone receptor-positive breast cancer.

Hormone therapy as an anticancer agent for hormone receptor-positive luminal type A breast cancer of the present invention preferably includes at least one selected from the group consisting of a selective estrogen receptor modulator (SERM), a selective estrogen receptor degrader (SERD), and an aromatase inhibitor (AI), and more preferably a selective estrogen receptor modulator (SERM) or a selective estrogen receptor degrader (SERD), which are ERα targeting agents.

The SERM includes, for example, tamoxifen, toremifene, raloxifene, bazedoxifene, ospemifene, droloxifene or iodoxifen.

The SERD includes, for example, fulvestrant, RAD 1901, ARN-810 (GDC0810), or AZD9496.

The AI includes, for example, exemestane, anastrozole, letrozole, vorozole, formestane or fadrozole.

As used herein, the term "comprising as an active ingredient" means that an inhibitor, which is an active ingredient of the present invention, is included in an amount sufficient to achieve a predetermined efficacy or activity. The HD AC 1/2 inhibitor and the BCL11A inhibitor can be administered in a pharmaceutically effective amount, and the effective dose level can be determined depending on the subject's type, age, gender, sensitivity to drugs, treatment period, concurrently used drugs and other medical factors.

The pharmaceutical composition according to one embodiment of the present invention may be administered as an individual therapeutic agent or in combination with other therapeutic agents and may be administered sequentially or simultaneously with existing therapeutic agents.

The daily dose of the pharmaceutical composition may be 0.01 to 500 mg/kg, and may be administered once or several times a day, but it can be easily determined by a person skilled in the art as an amount capable of obtaining the maximum effect without side effects by considering the patient's weight, age, gender, health condition, diet, administration time, administration method, excretion rate, severity of disease, etc.

The pharmaceutical composition may be administered by various routes such as oral, subcutaneous, intraperitoneal, intrapulmonary, intranasal, intramuscular, intravenous, and arterial.

Further, the pharmaceutical composition of the present invention may be formulated by including pharmaceutically acceptable carriers, excipients and/or diluents in addition to the above active ingredients.

The pharmaceutical preparations may be formulated in the form of oral formulations such as powders, granules, tablets, coated tablets, capsules, suspensions, emulsions, syrups, suppositories, aerosols, external preparations, suppositories or injections, but are limited thereto.

The pharmaceutical preparation may be prepared by further mixing one or more excipients, for example, starch, lactose, gelatin, sucrose, lubricant, preservative, flavoring agent, sweetener, and the like.

The composition of the present invention may contain at least one known active ingredient having a preventive or therapeutic effect on breast cancer, a target of the present invention, together with an HDAC1/2 inhibitor, a BCL11A inhibitor, and an anticancer agent.

The composition of the present invention may further include a pharmaceutically acceptable additive, in which the pharmaceutically acceptable additive includes starch, gelatinized starch, microcrystalline cellulose, lactose, povidone, colloidal silicon dioxide, calcium hydrogen phosphate, lactose, mannitol, taffy, gum arabic, pregelatinized starch, corn starch, powdered cellulose, hydroxypropyl cellulose, opadry, sodium starch glycolate, carnauba wax, synthetic aluminum silicate, stearic acid, magnesium stearate, aluminum stearate, calcium stearate, white sugar, and the like. The pharmaceutically acceptable additive according to the present invention is preferably included in an amount of 0.1 parts by weight to 90 parts by weight based on the composition, but is not limited thereto.

The composition of the present invention can be administered in various oral or parenteral dosage forms during actual clinical administration, formulations can be prepared using diluents or excipients such as commonly used fillers, extenders, binders, wetting agents, disintegrants, and surfactants, and suitable formulations known in the art are preferably those disclosed in the literature (Remington's Pharmaceutical Science, recently, Mack Publishing Company, Easton PA).

Solid formulations for oral administration include tablets, pills, powders, granules, capsules, etc., and these solid formulations are prepared by mixing at least one or more excipients, for example, starch, calcium carbonate, sucrose or lactose, gelatin, and the like. In addition to simple excipients, lubricants such as magnesium stearate and talc are also used. Further, the liquid formulations for oral administration include suspensions, solutions for oral administration, emulsions, syrups, etc. and may contain various excipients, such as wetting agents, sweeteners, aromatics, and preservatives, in addition to water and liquid paraffin, which are commonly used simple diluents.

The formulations for parenteral administration include sterilized aqueous solutions, non-aqueous solutions, suspensions, emulsions, freeze-dried formulations, and suppositories. Propylene glycol, polyethylene glycol, vegetable oils such as olive oil, and injectable esters such as ethyl oleate may be used as non-aqueous solvents and suspending agents. Witepsol, macrogol, tween 61, cacao butter, laurin paper, glycerogeratin, and the like may be used as a base for the suppository.

The dosage of the pharmaceutical composition of the present invention may vary depending on the formulation method, administration method, administration time and/or administration route of the pharmaceutical composition and may vary according to several factors, including the type and degree of response to be achieved by administration of the pharmaceutical composition, the to-be-administered subject's type, age, weight, general health condition, symptom or degree of disease, gender, diet, excretion, and components of drug or other compositions used simultaneously or separately in the subject and similar factors well known in the medical field, and a person of ordinary skill in the art can readily determine and prescribe an effective dosage for the desired treatment.

The pharmaceutical composition of the present invention is preferably administered at a concentration of, for example, 0.01 to 500 mg/kg, but the dosage does not limit the scope of the present invention in any way.

The administration route and administration method of the pharmaceutical composition of the present invention may be independent but are not particularly limited in the method, and any administration route and administration method may be followed as long as the pharmaceutical composition can reach the target site.

The pharmaceutical composition may be administered orally or parenterally. The parenteral administration method includes, for example, intravenous administration, intraperitoneal administration, intramuscular administration, transdermal administration, subcutaneous administration, or the like.

The pharmaceutical composition of the present invention may be used alone or in combination with methods using surgery, radiation therapy, hormone therapy, chemotherapy, and biological response modifiers for the prevention or treatment of target indications.

According to another aspect of the present invention, the present invention may provide a food composition for preventing or alleviating ER-negative breast cancer, the composition including the BCL11A inhibitor and the HDAC1/2 inhibitor as active ingredients, and a health functional food including the same.

As used herein, the term "health functional food" refers to food manufactured and processed using raw materials or ingredients having useful functionalities for the human body. Here, 'functional' refers to intake for the purpose of adjusting nutrients for the structure and function of the human body or obtaining useful effects for health purposes such as physiological functions.

The food composition includes all forms such as functional food, nutritional supplement, health food, and food additives. The above types may be prepared in various forms according to conventional methods known in the art.

The food composition may be formulated in the same way as the pharmaceutical composition and used as a health functional food and may be added to various foods.

As a specific example, when preparing food or beverage, the composition of the present invention is added in an amount of 15% by weight or less, preferably 10% by weight or less, based on the raw material. However, for long-term intake for health and hygiene purposes or for health control purposes, it may be added in an amount below the above range, and since there is no problem in terms of safety, the active ingredient may be used in an amount above the above range. The type of food is not particularly limited, but examples of food to which cucurbitacin D of the present invention can be added include meat, sausage, bread, chocolate, candy, snacks, confectionery, pizza, ramen, other noodles, chewing gum, dairy products including ice cream, various soups, beverages, tea, drinks, alcoholic beverages, vitamin complexes, etc., and include all health foods in a conventional sense.

When the composition of the present invention is prepared as a beverage, it may contain additional components such as various flavoring agents or natural carbohydrates, like conventional beverages. The natural carbohydrates may include monosaccharides such as glucose and fructose, disaccharides such as maltose and sucrose, natural sweeteners such as dextrin and cyclodextrin, and synthetic sweeteners such as saccharin and aspartame. The natural carbohydrate is included in an amount of 0.01% by weight to 10% by weight, preferably 0.01% by weight to 0.1% by weight, based on the total weight of the food composition of the present invention.

The food composition of the present invention may include various nutrients, vitamins, electrolytes, flavors, colorants, pectic acid and its salts, alginic acid and its salts, organic acids, protective colloidal thickeners, pH adjusters, stabilizers, preservatives, glycerin, alcohol, a carbonation agent used in and carbonated beverages, and may include fruit flesh for the manufacture of natural fruit juice, fruit juice beverages and vegetable beverages but is not limited thereto. These components may be used independently or in combination. The proportion of the above additive is not particularly limited, but the additive is preferably included within the range of 0.01% by weight to 0.1% by weight based on the total weight of the food composition of the present invention.

Further, according to another aspect of the present invention, the present invention provides a screening method for a drug for enhancing sensitivity to an anticancer agent for ER-negative breast cancer, the method including the following steps:
(a) treating an isolated ER-negative breast cancer cell with a candidate material;
(b) measuring the expression levels of BCL11A and HDAC1/2 in ER-negative breast cancer cells treated with the candidate material; and
(c) determining that the candidate material can be used as a drug for enhancing sensitivity to anticancer agent for the treatment of ER-negative breast cancer when the expression levels of BCL11A and HDAC1/2 measured in step (b) are lower than those of the isolated malignant breast cancer cells that are not treated with the candidate material.

The drug for enhancing sensitivity to anticancer agent refers to a substance that increases the sensitivity of tumor cells to an anticancer agent as a sensitizer or helps the anticancer agent to act specifically on tumor cells to achieve a therapeutic effect with a small dose.

The term "candidate material" used while referring to the screening method of the present invention refers to an unknown test substance used in screening to examine whether or not it affects the expression level of a gene or affects the expression or activity of a protein. The candidate materials include, for example, compounds, nucleotides, proteins, antibodies, and natural product extracts, but are not limited thereto.

The assay for measuring the expression levels of BCL11A and HDAC1/2 is for detecting the BCL11A and HDAC1/2 mRNA, and it may include, but is not limited to, at least one selected from the group consisting of reverse transcription polymerase reaction, competitive reverse transcription polymerase reaction, real-time reverse transcription polymerase reaction, RNase protection assay (RPA), northern blotting, and DNA chip.

The assay for measuring the expression levels of BCL11A and HDAC1/2 is for detecting the BCL11A and HDAC1/2 proteins, and it may include, but is not limited to, at least one selected from the group consisting of western blotting, enzyme-linked immunosorbent assay (ELISA), enzyme-linked lectin assay (ELLA), radiation immunoassay, radiation immunodiffusion, Ouchterlony immunodiffusion, rocket immunoelectrophoresis, immunohistochemical staining, immunoprecipitation assay, complement fixation assay, FACS, and protein chip.

In the screening method for a drug for enhancing the sensitivity to an anticancer agent for ER-negative breast cancer of the present invention, when the expression of BCL11A and HDAC1/2 is reduced by treatment with a candidate material, ER-negative breast cancer is transdifferentiated into luminal A-type breast cancer to increase the sensitivity of tumor cells to drugs for treating ER-negative breast cancer (e.g., anticancer agent), so it may be determined that the candidate material can be used as a drug for enhancing sensitivity to anticancer agent for treating ER-negative breast cancer. In this screening method, the activity measurement may be easily determined according to the expression levels of BCL11A and HDAC1/2.

Since the method of the present invention uses the expression levels of the above-described BCL11A and HDAC1/2, redundant descriptions thereof are excluded to avoid excessive complexity in the present specification.

### [Advantageous Effects]

According to the present invention, when the target genes of the present invention are inhibited, BLT is induced to transdifferentiate basal-like or triple-negative breast cancer into a subtype of luminal A-type breast cancer that responds to anticancer therapy, that is, is compatible with hormone therapy, and thus these may be effective and new drug therapies that have not been tried before, realize personalized medicine, increase the survival rate of patients through targeted therapy, and contribute to preventing deterioration in quality of life due to unnecessary anticancer drug treatment.

### [Description of Drawings]

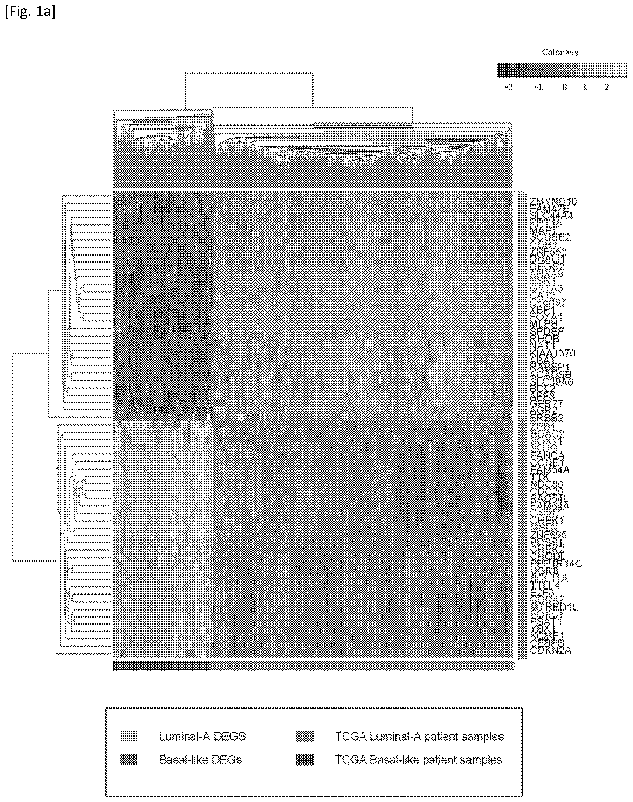

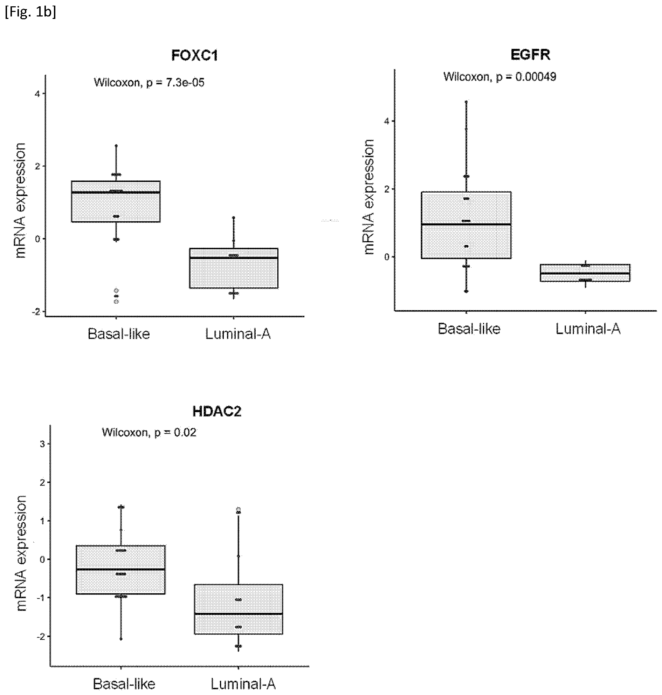

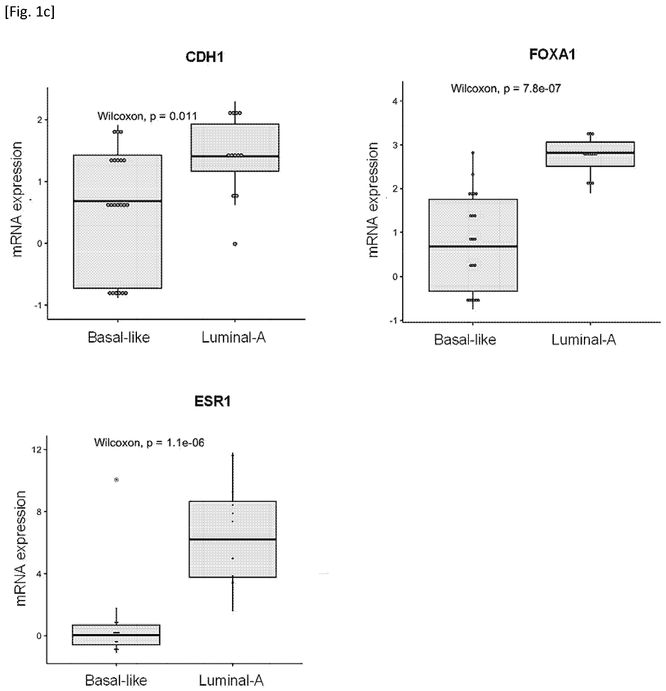

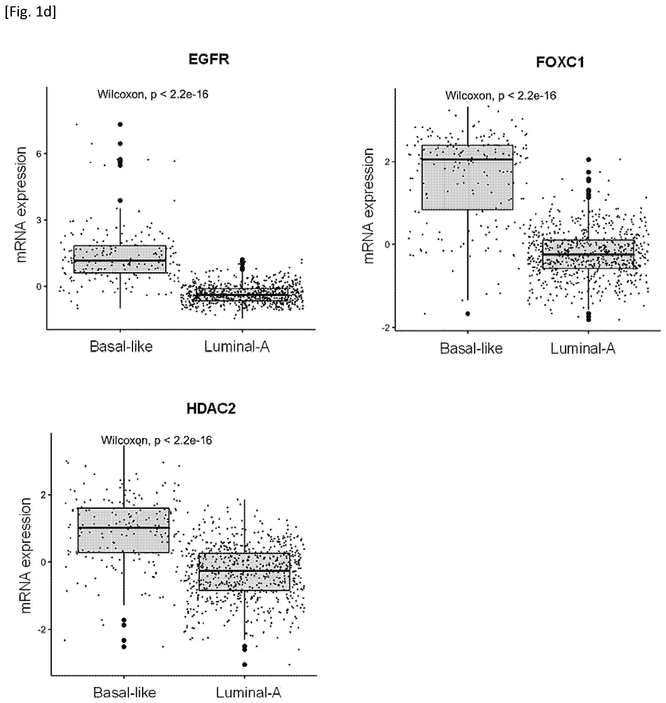

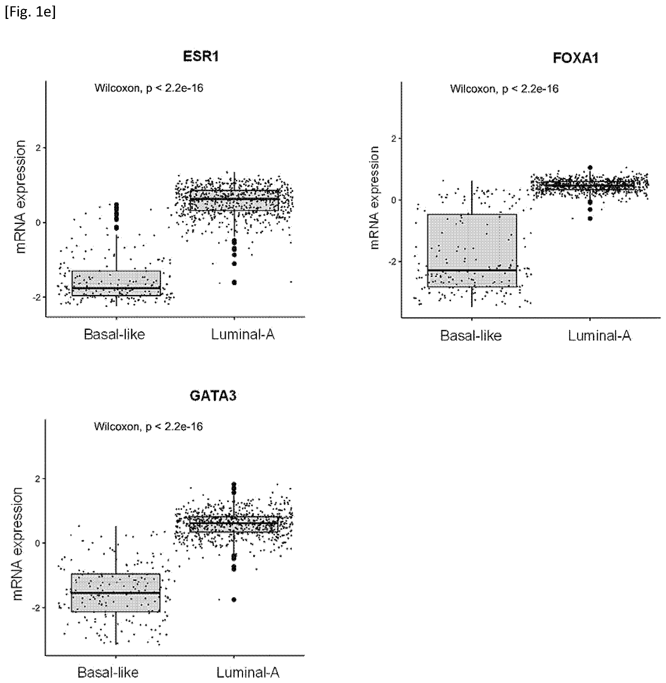

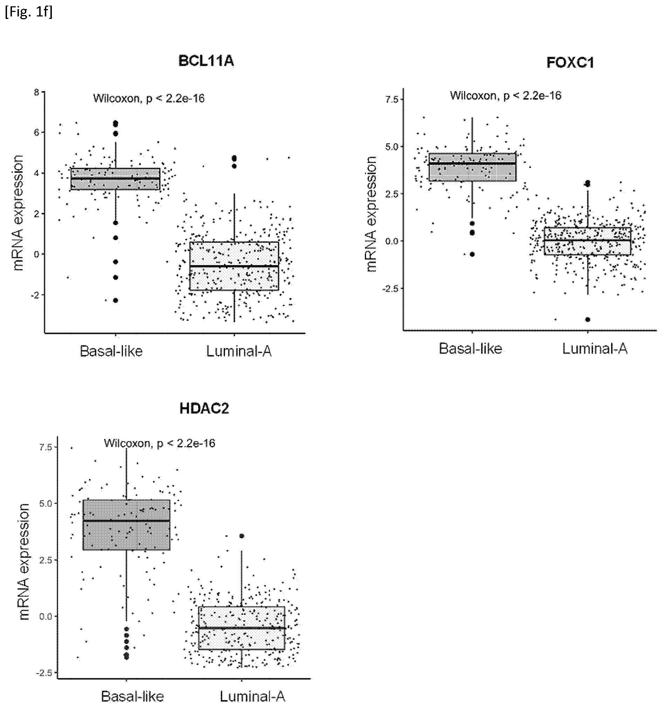

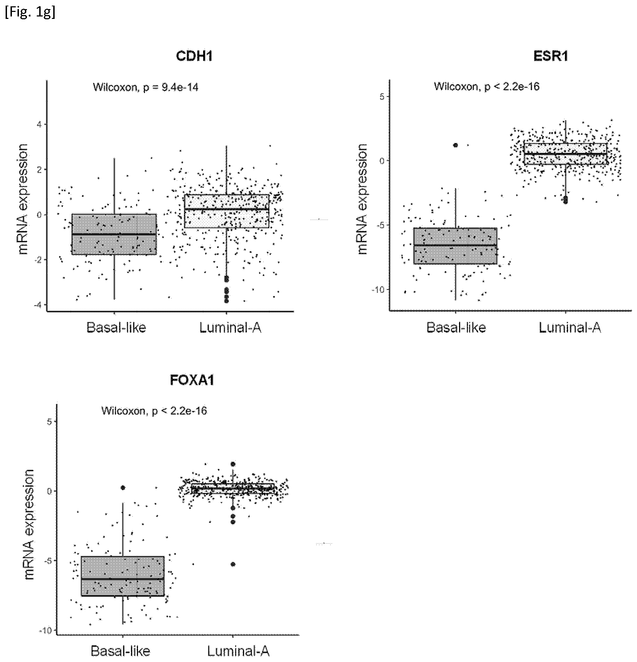

FIG. 1 shows the DEG analysis results for constructing the BLT network model. FIG. 1A shows a relatively highly expressed gene group for each type using The Cancer Genome Atlas (TCGA) data. Among them, genes with the greatest expression difference (red) were selected and included in the network as marker genes for each triple-negative breast cancer and luminal type A. FIG. 1B shows as a box graph whether the triple-negative breast cancer genes among the selected genes are expressed higher in the triple-negative breast cancer cell line than in the luminal type A cell line even in the Cancer Cell Line Encyclopedia (CCLE) data. FIG. 1C shows as a box graph whether luminal type A genes among the selected genes are expressed higher in the luminal type A cell line than in the triple-negative breast cancer cell line even in the CCLE data. Similarly, FIGS. 1D and 1E are verified through Molecular Taxonomy of Breast Cancer International Consortium (METABRIC) data, and FIGS. 1F and 1G are verified through TCGA data.

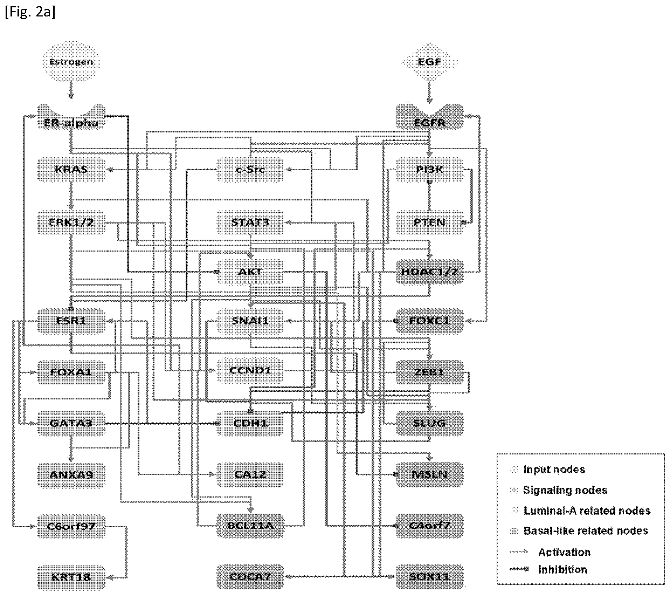

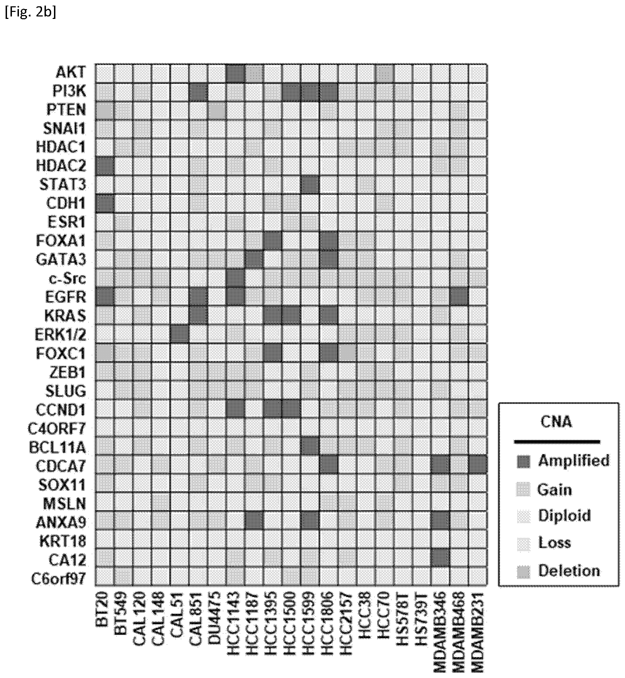

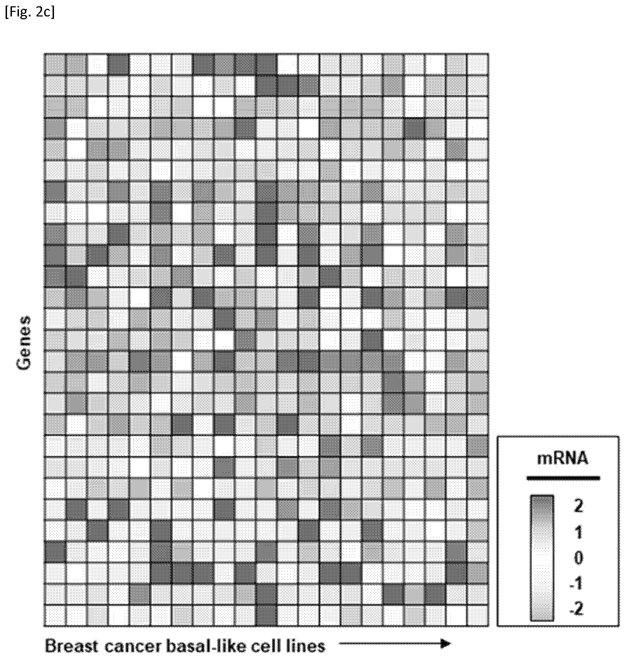

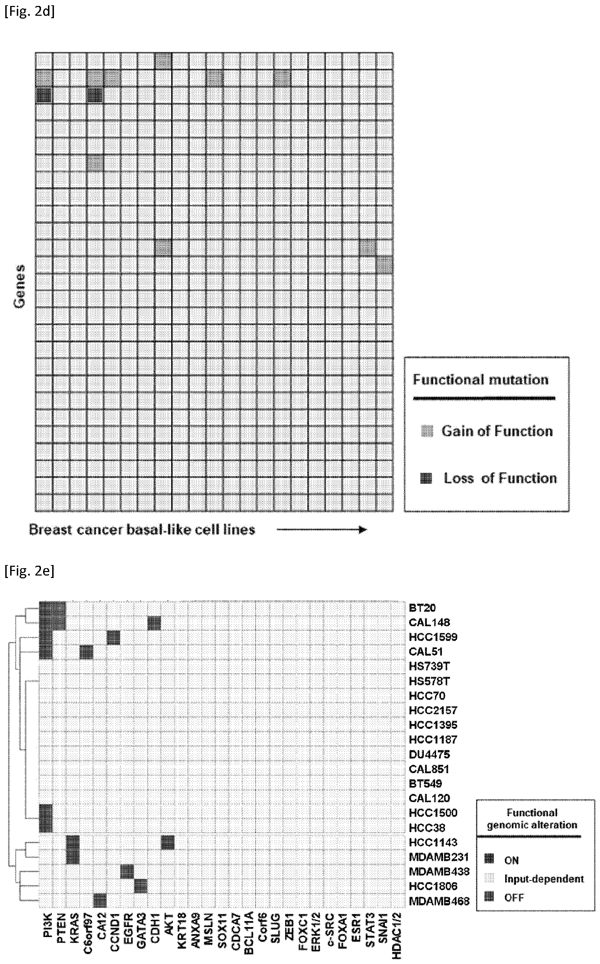

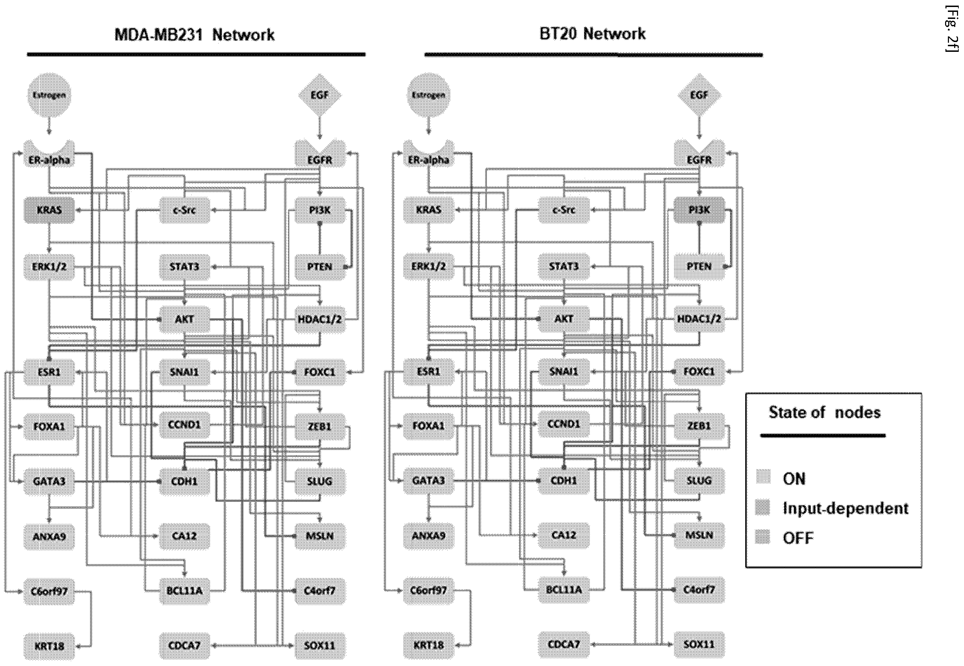

FIG. 2 shows a cell line-specific BLT network model created by mapping genomic information of a basal-like cell line. FIG. 2A shows the completed network. FIG. 2B shows a network mapped using CNA, FIG. 2C shows a network mapped using RNA expression, and FIG. 2D shows a network mapped using mutation information. FIG. 2E shows a network in which a scale called functional genomic alternation is created using all of the above genomic information and the information is mapped. FIG. 2F shows a cell line network created by mapping the genome information of the cell line to be used in each experiment.

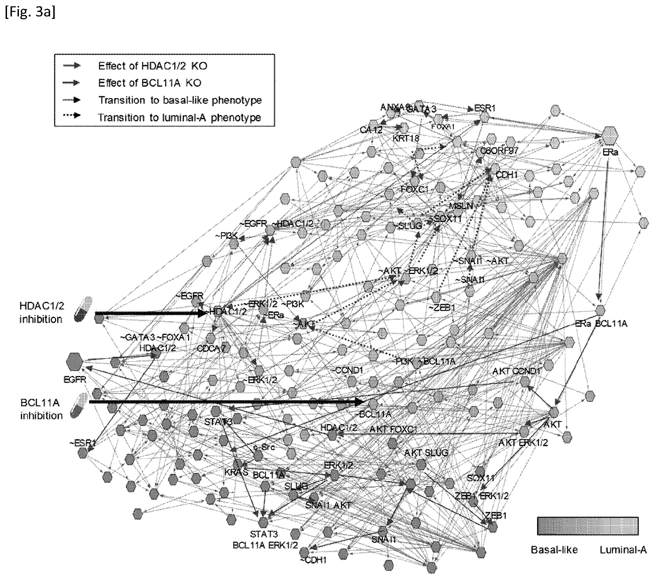

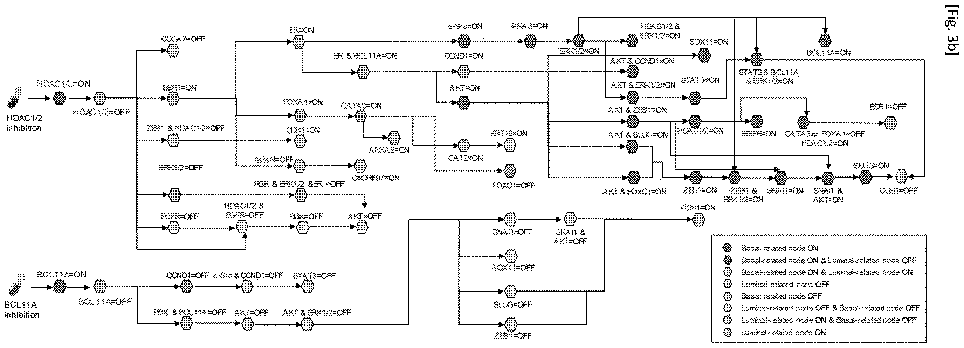

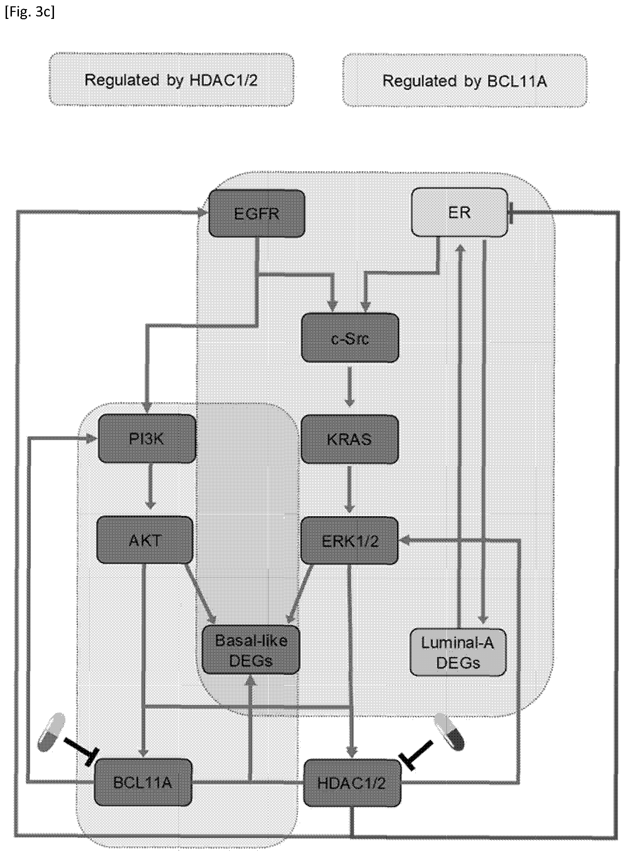

FIG. 3 shows the mechanism of BLT induced by BCL11A KO and HDAC1/2 KO. FIG. 3A shows the state change of other genes in the network when the found gene is controlled. FIG. 3B shows the mechanism of major genes among the changes shown in FIG. 3A. FIG. 3C shows the control mechanism of the signaling pathway when each gene is controlled based on the mechanism.

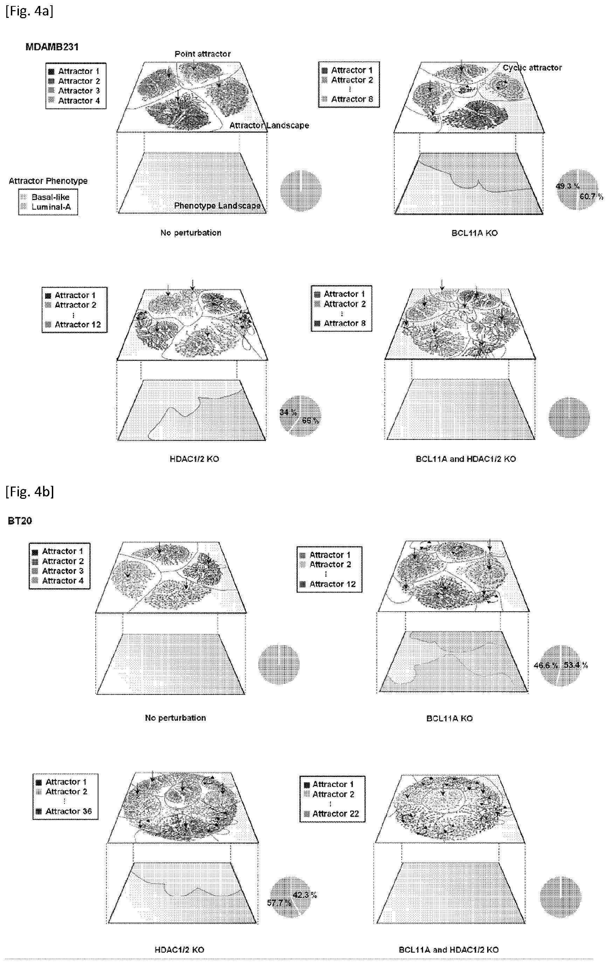

FIG. 4 shows the dynamic stability of modified attractors after KOs (knockout) of BCL11A and HDAC1/2. FIG. 4A shows the phenotypic ratio of each attractor when each gene was controlled in the MDAMB231 cell line. FIG. 4B shows the phenotypic ratio of each attractor when each gene was controlled in the BT20 cell line.

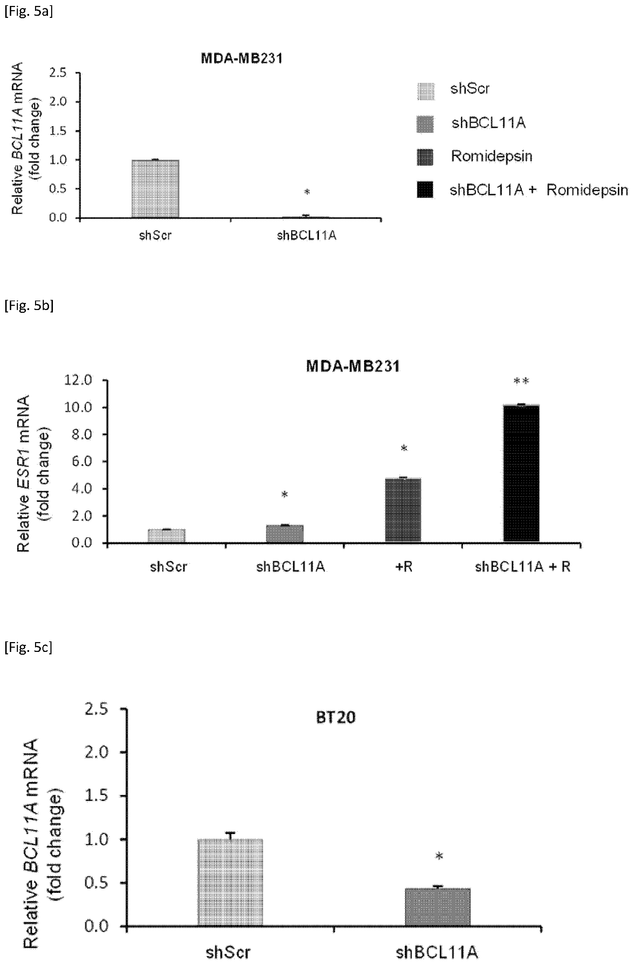

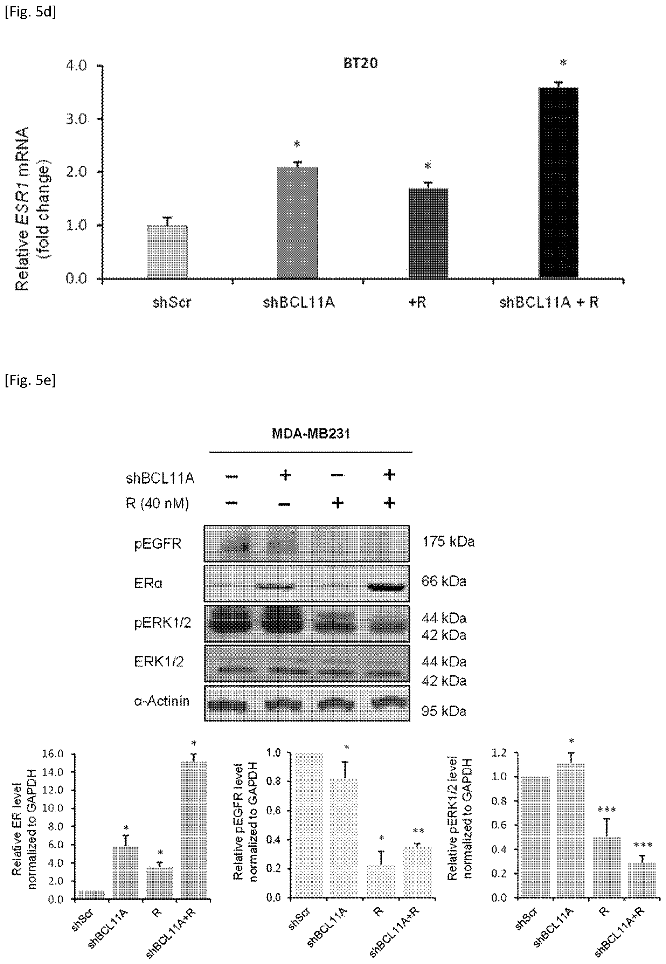

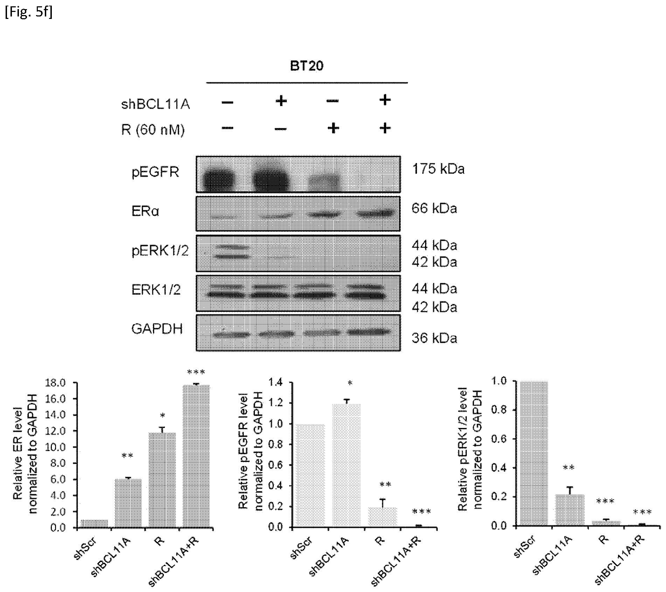

FIG. 5 shows the effects of BCL11A and HDAC1/2 KO on the expression of ESR1 mRNA and ERα. FIG. 5A shows the result of knocking down the BCL11A gene using shRNA in the MDAMB231 cell line to reduce its expression. FIG. 5B shows changes in ESR1 gene expression after controlling the expression of each target gene in the MDAMB231 cell line. FIG. 5C shows the results of knocking down the BCL11A gene using shRNA in the BT20 cell line to reduce its expression. FIG. 5D shows changes in ESR1 gene expression after controlling the expression of each target gene in the BT20 cell line. FIG. 5E shows the results of marker protein changes in ERa and triple-negative breast cells after controlling the expression of each target gene in the MDAMB231 cell line. FIG. 5F shows the results of marker protein changes in ERa and triple-negative breast cells after controlling the expression of each target gene in the BT20 cell line.

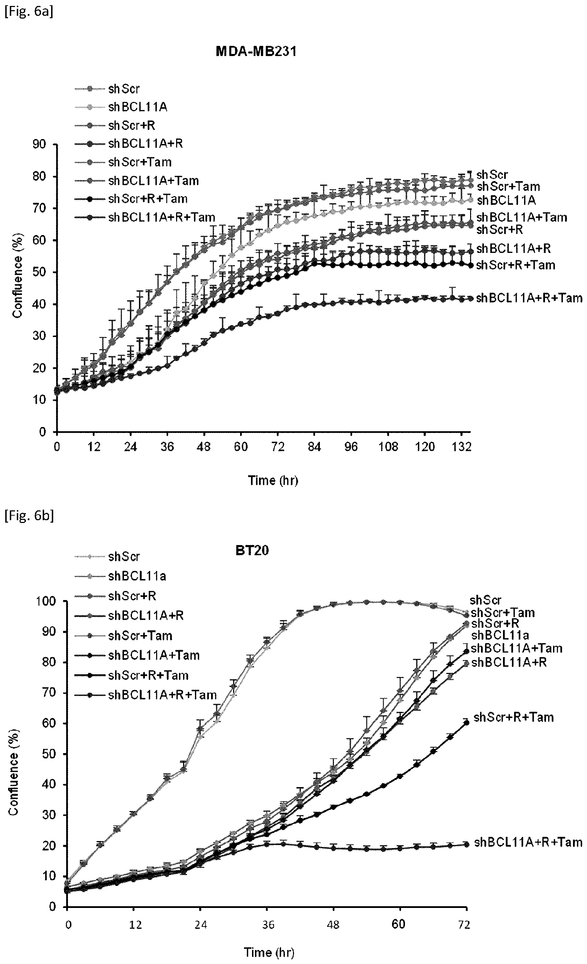

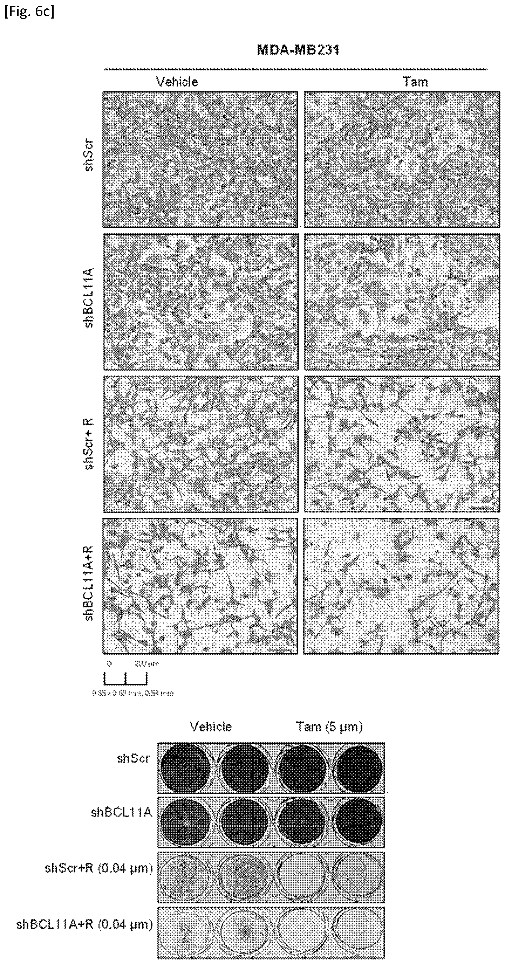

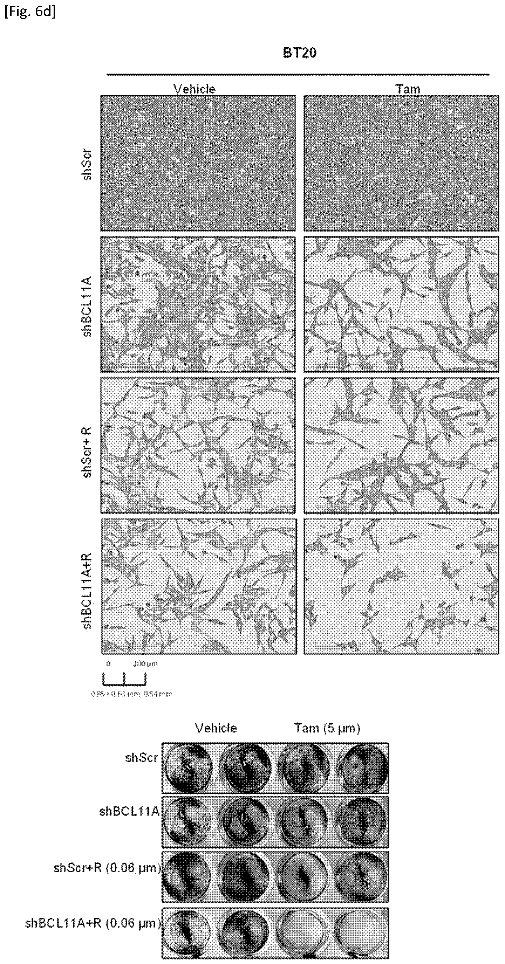

FIG. 6 shows the results of the tamoxifen drug response after BCL11A and HD AC 1/2 KO. FIG. 6A shows the response of the cells to the tamoxifen drug as a growth rate after controlling the expression of each target gene in the MDAMB231 cell line. FIG. 6B shows the response of the cells to the tamoxifen drug as a growth rate after controlling the expression of each target gene in the BT20 cell line. FIG. 6C shows changes in cells in the tamoxifen drug response for each condition in the MDAMB231 cell line. FIG. 6D shows changes in cells in the tamoxifen drug response for each condition in the BT20 cell line.

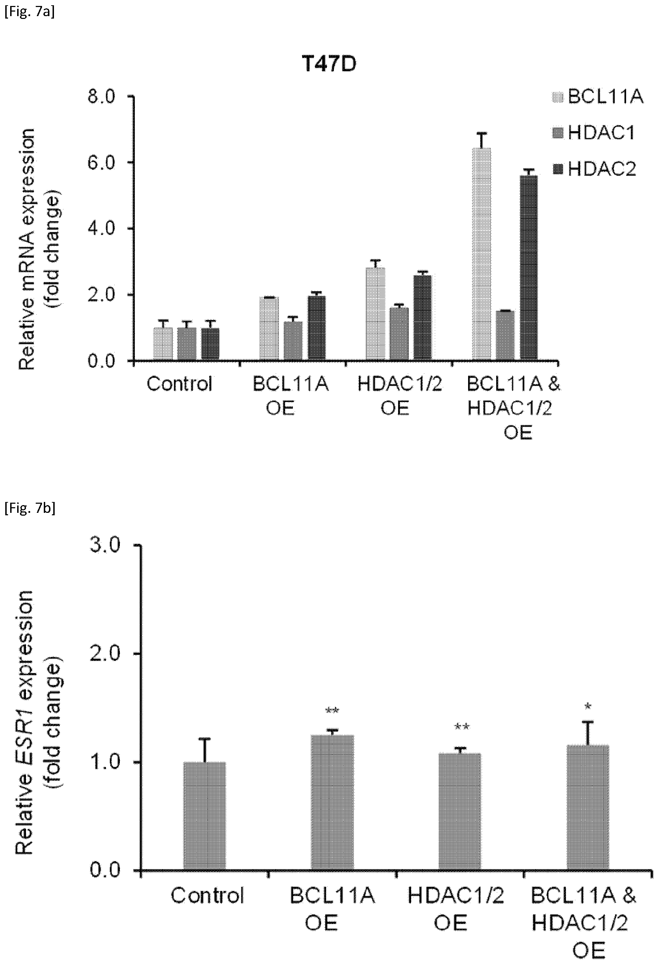

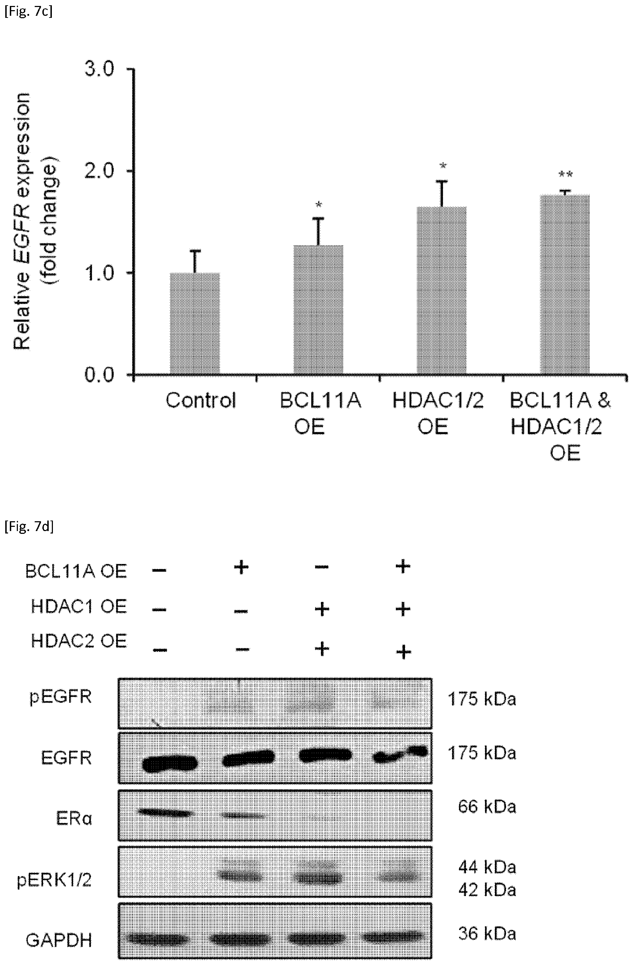

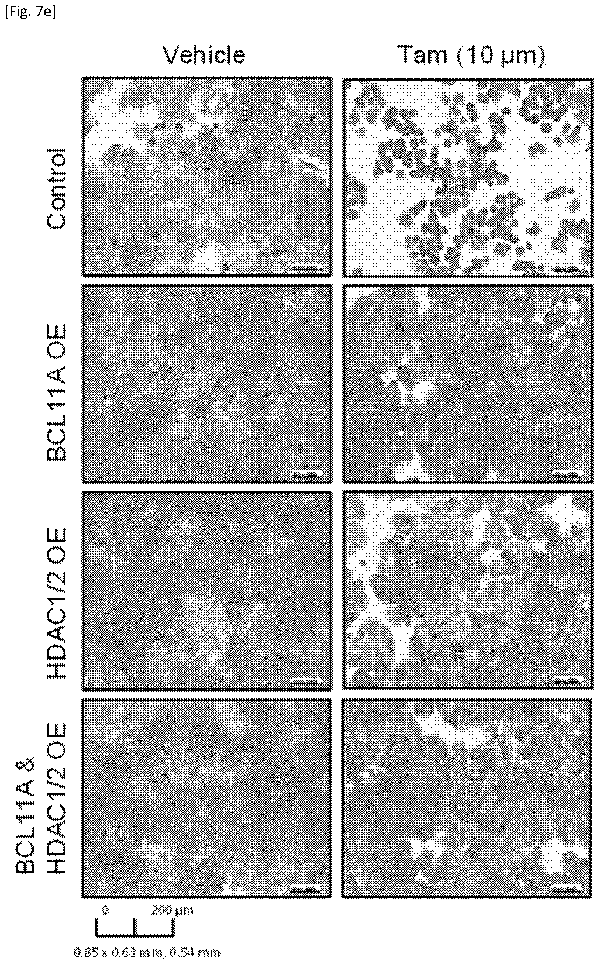

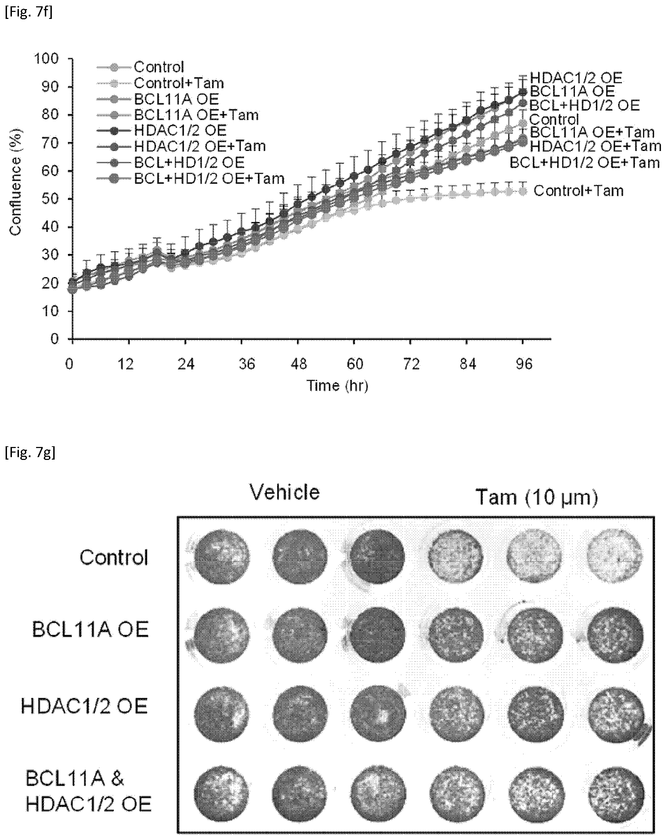

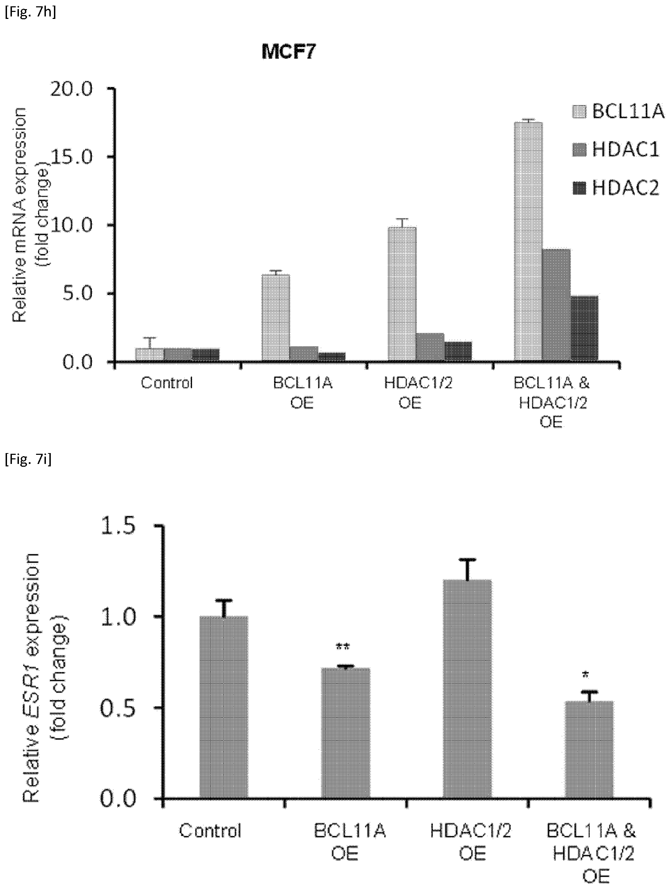

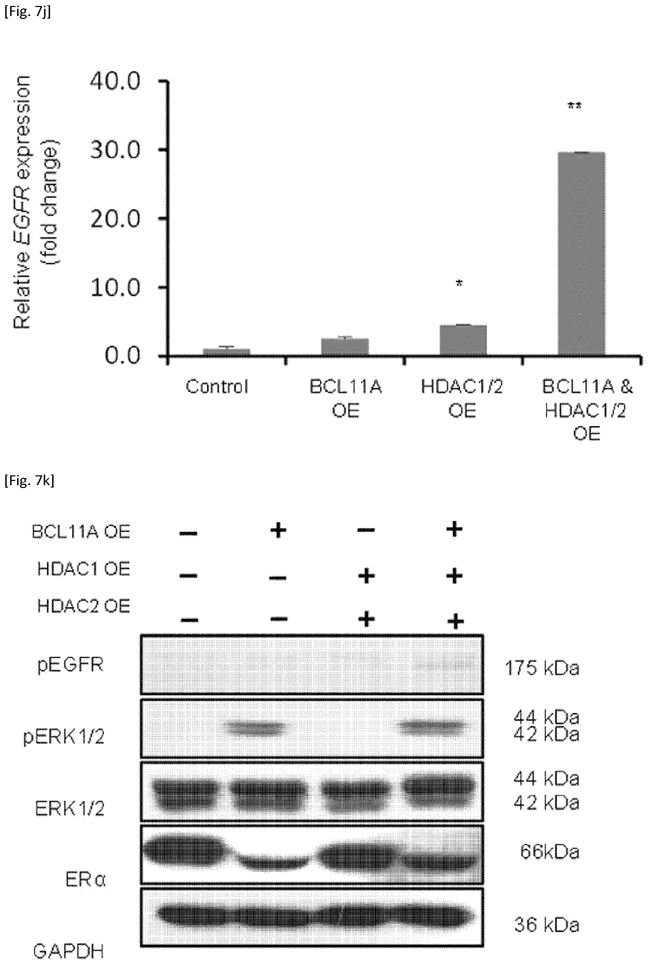

FIG. 7 shows the results of the tamoxifen drug response after BCL11A and HD AC 1/2 overexpression. FIG. 7A shows the results of overexpressing each target gene in the T47D cell line as a graph. FIG. 7B shows the mRNA change of ESR1 after overexpression. FIG. 7C shows the mRNA change of EGFR after overexpression. FIG. 7D shows the results of marker protein changes in ERa and triple-negative breast cells after overexpression. FIG. 7E shows changes in cells in the tamoxifen drug response for each condition. FIG. 7F shows the response of cells to the tamoxifen drug as a growth rate after controlling the expression of each target gene in BT20. FIG. 7G shows the response of the cells to the tamoxifen drug by staining the cells. FIGS. 7H to 7K show verification that mRNA and protein expression changes are similar by applying the same experiment to the MCF7 cell line.

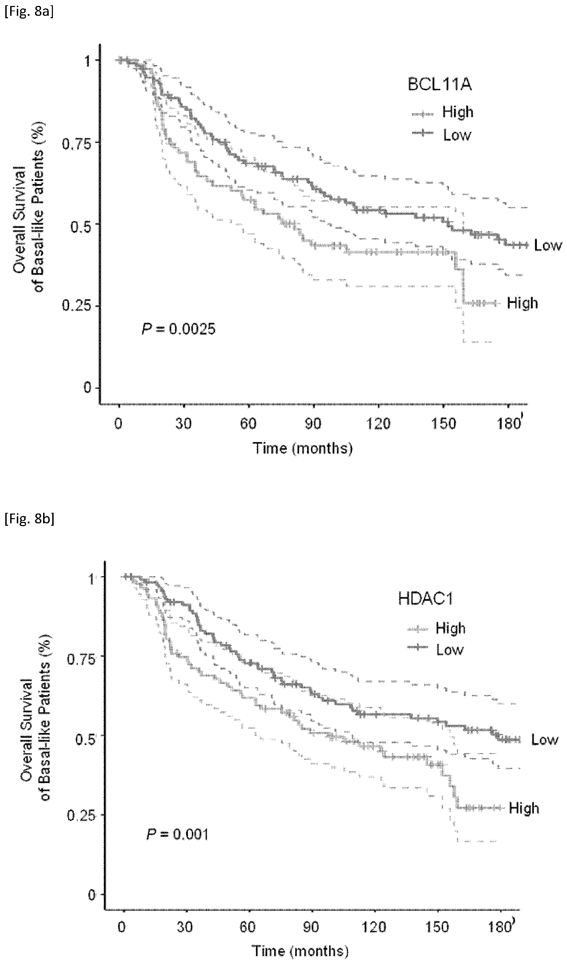

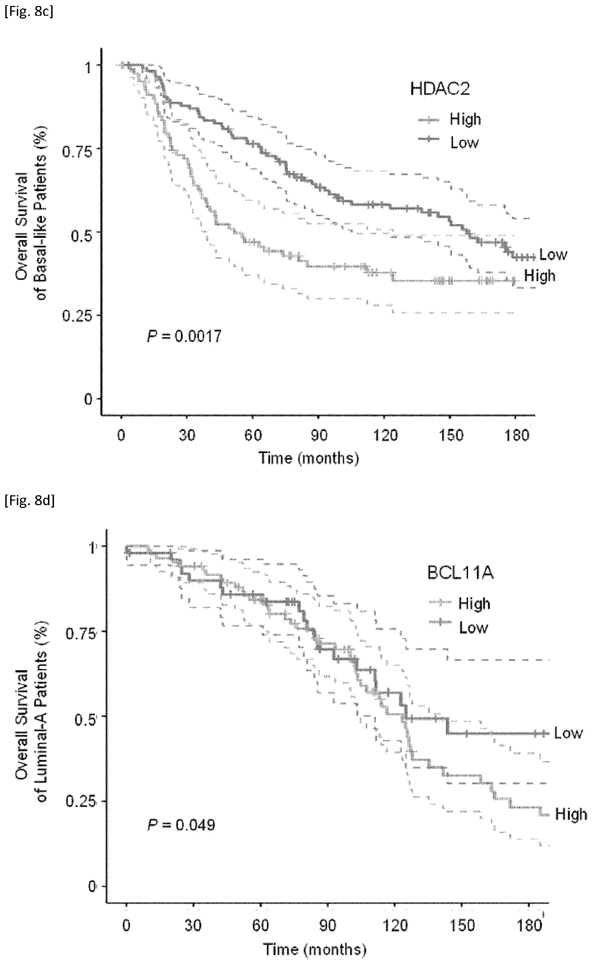

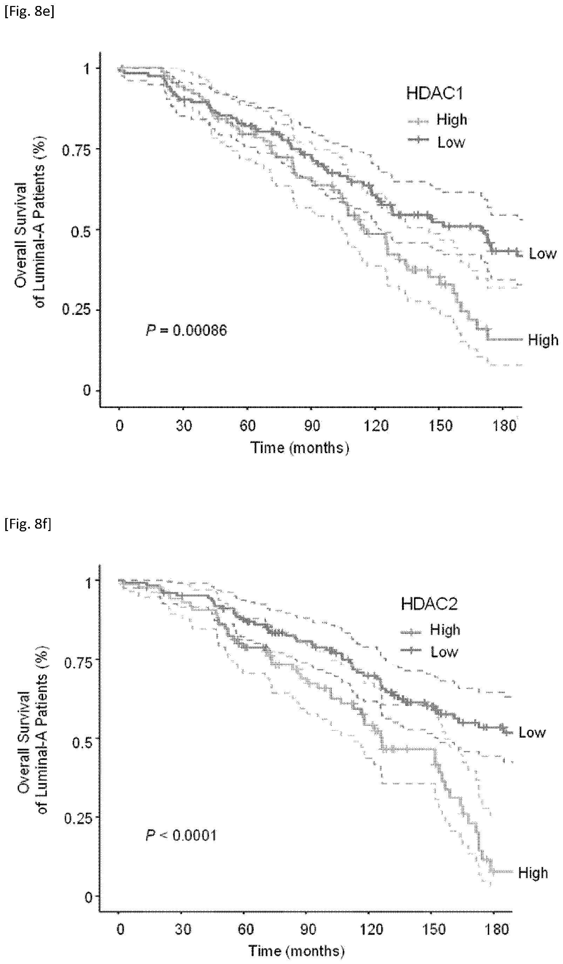

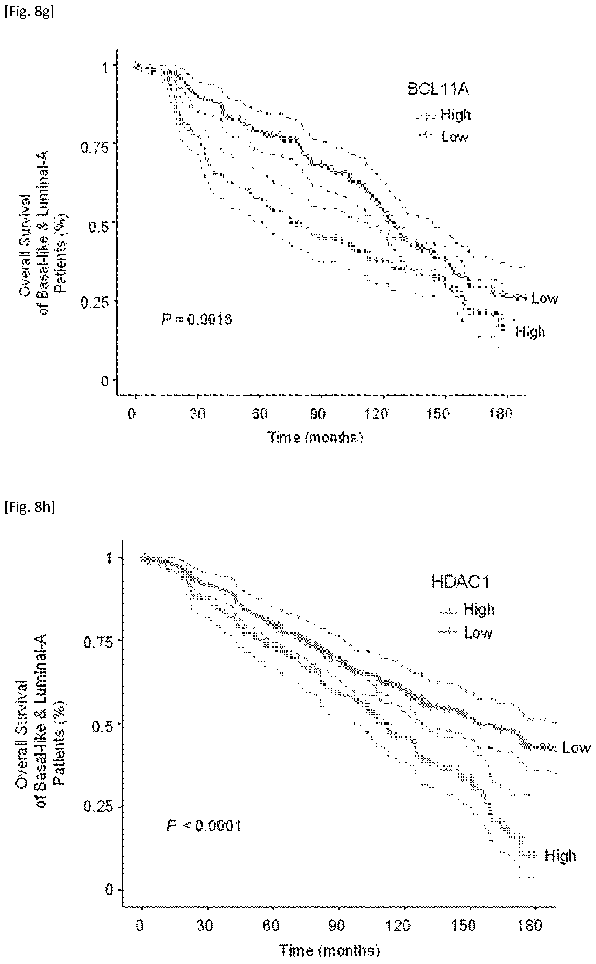

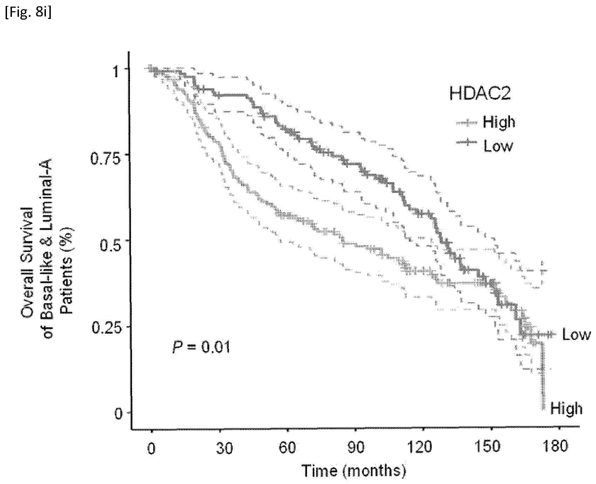

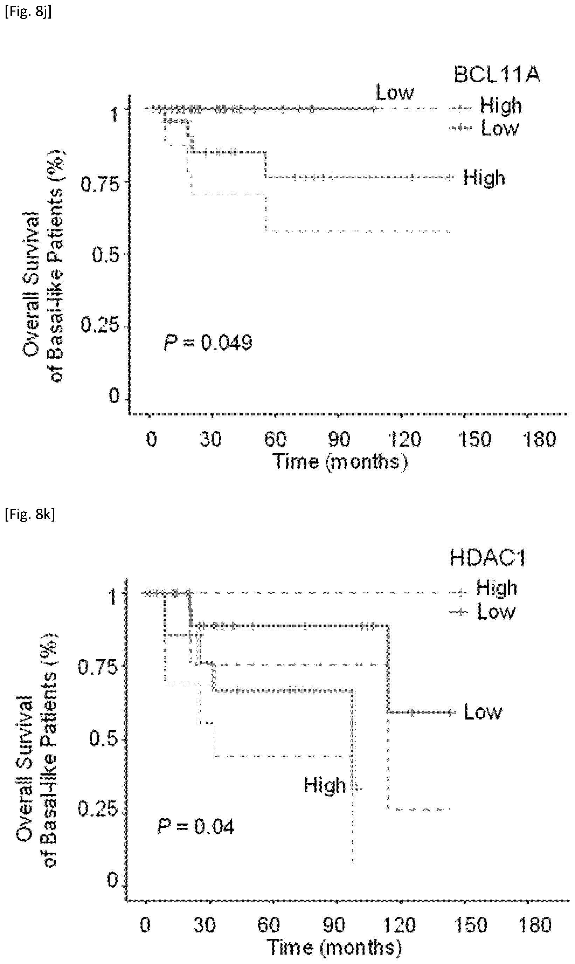

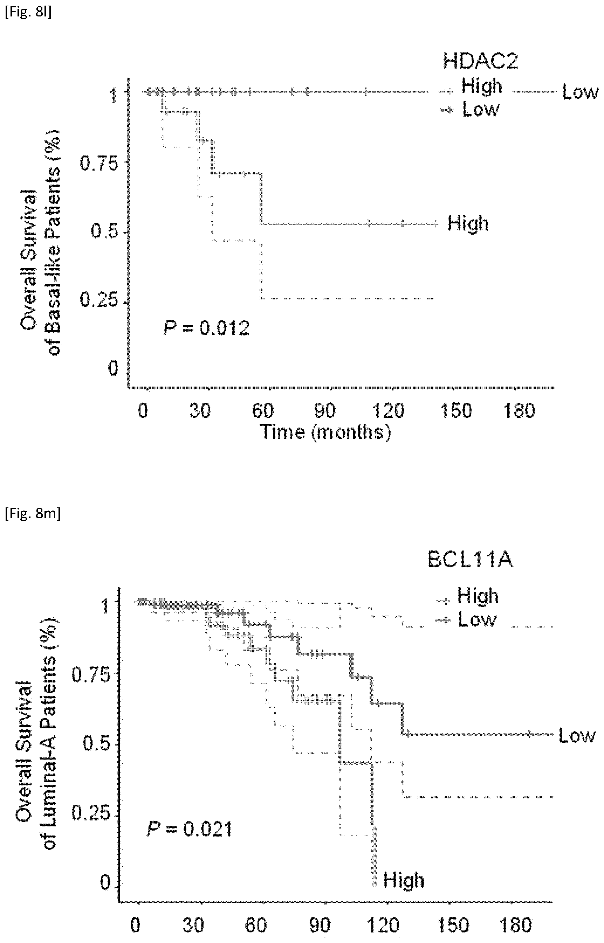

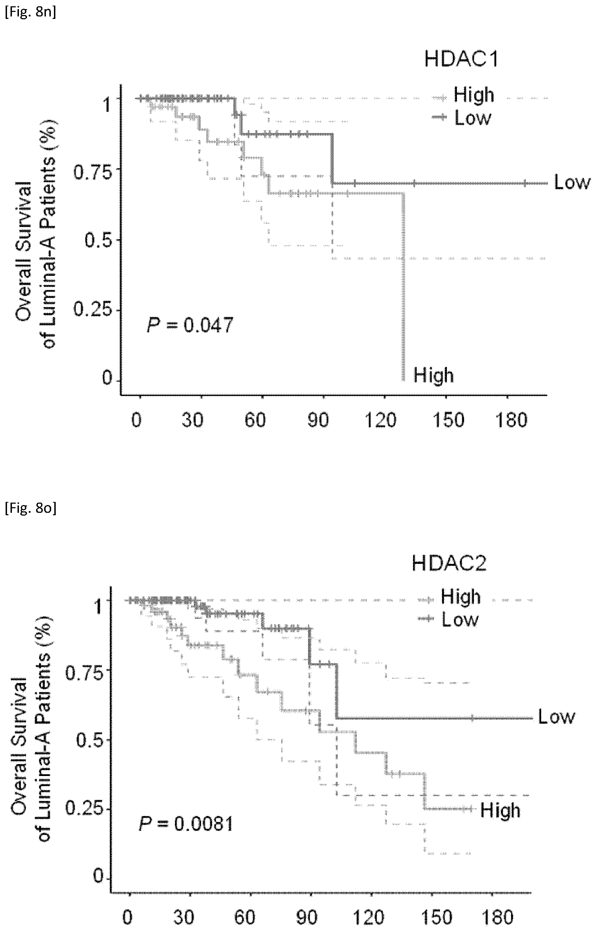

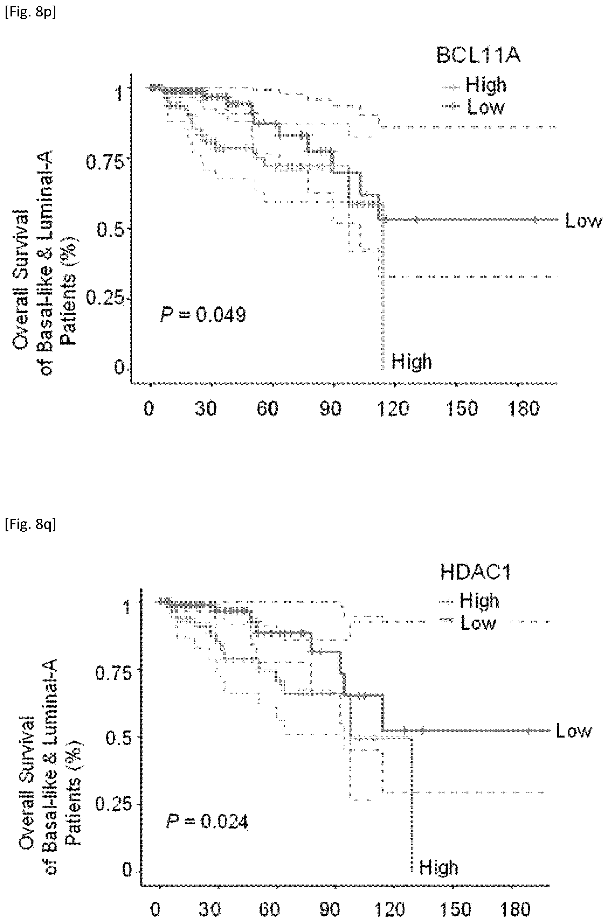

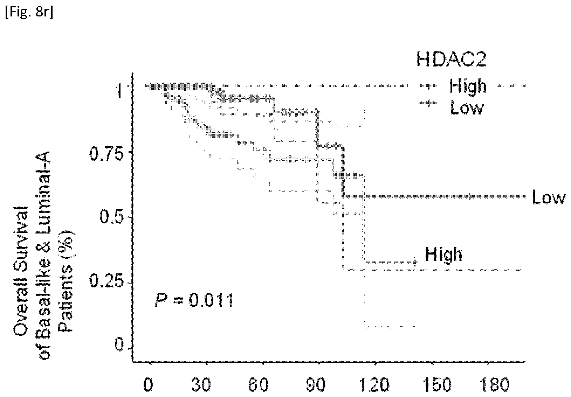

FIG. 8 shows the result of analyzing the survival rate of patients according to BCL11A and HDAC1/2 expression. FIGS. 8A to 8C show results of survival rate analysis according to high and low expression of each gene in METABRIC triple-negative breast cancer patients. FIGS. 8D to 8F show results of survival rate analysis according to high and low expression of each gene in METABRIC luminal type A patients. FIGS. 8G to 8I show results of survival rate analysis according to high and low expression of each gene in two METABRIC patient groups. FIGS. 8J to 8L show results of survival rate analysis according to high and low expression of each gene in TCGA triple-negative breast cancer patients. FIGS. 8M to 8O show results of survival rate analysis according to high and low expression of each gene in TCGA luminal type A patients. FIGS. 8P to 8R show results of survival rate analysis according to high and low expression of each gene in two TCGA patient groups.

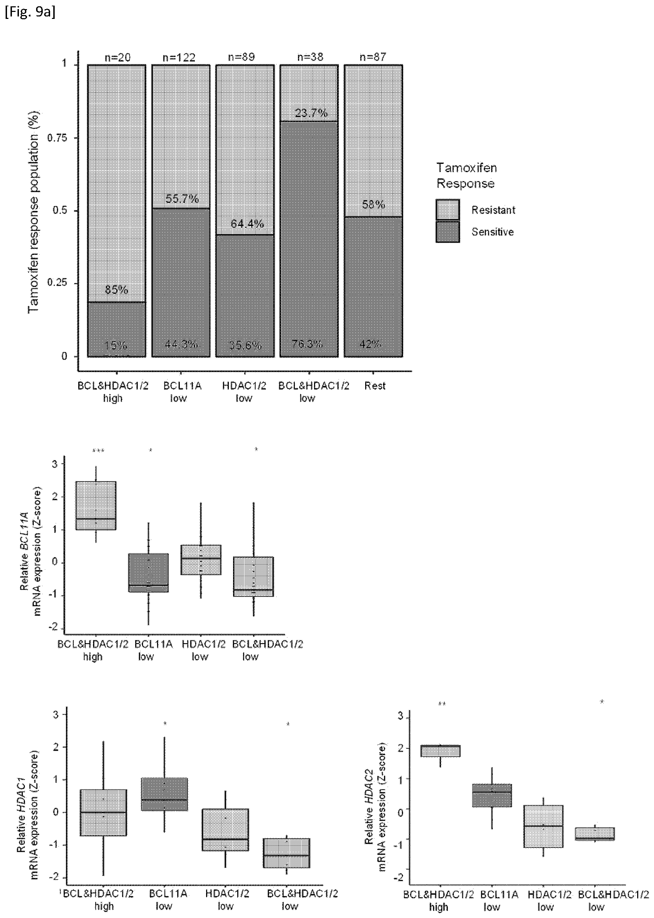

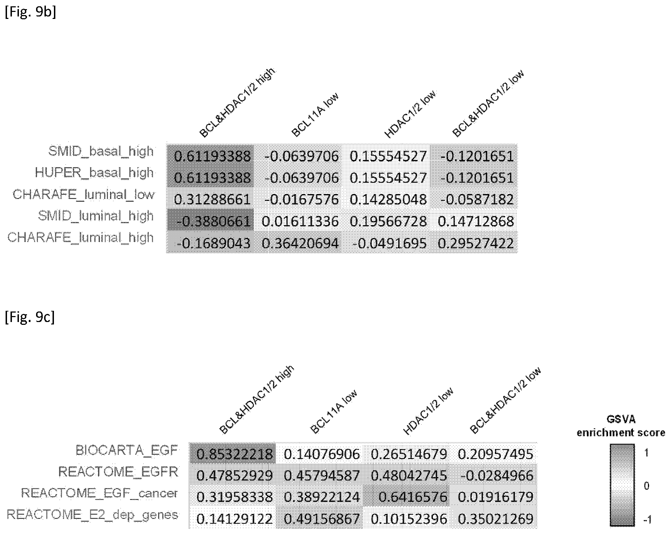

FIG. 9 shows the results of the patient's tamoxifen drug response according to BCL11A and HDAC1/2 expression. FIG. 9A shows the results of the drug response to tamoxifen after dividing the patient groups according to each gene expression as a bar graph. FIGS. 9B to 9C show, through GSVA analysis, that the gene expression pattern of each divided patient group approaches the gene expression pattern of the luminal type A when all of the target genes are inhibited.

### [Modes of the Invention]

Hereinafter, it is apparent to those skilled in the art that the Examples are only for explaining the present invention in more detail, and the scope of the present invention is not limited by these Examples according to the gist of the present invention.

### Example 1. DEG analysis to construct basal-to-luminal transition (BLT) network model

The present inventors constructed a BLT network model to explore molecular regulatory interactions during the basal-to-luminal transition (BLT) from basal-like breast cancer or triple-negative breast cancer type into luminal type by targeting basal-like breast cancer and triple-negative breast cancer (TNBC) types, which are malignant breast cancer types that have ER-negative and HER2-negative in common.

First, through the analysis of multiple breast cancer patients and cell line data (TCGA, CCLE, METABRIC), genes with high expression were extracted from basal-like breast cancer and luminal A-type breast cancer, which are clinically determined to be the same as triple-negative breast cancer in the art, and a mathematical model for BLT was built. The network model constructed by the present inventors used Kyoto Encyclopedia of Genes and Genomes (KEGG) based on the ERα and EGFR signaling pathway, which are pathways that play an important role in cancer cell growth, survival and tumorigenesis in luminal type A and basal-like breast cancer. Further, differentially expressed genes (DEGs) were used as additional phenotypic markers for each type.

To this end, the present inventors compared mRNA expression profiles of patients with basal-like breast cancer and luminal type A breast cancer of The Cancer Genome Atlas (TCGA) and identified DEGs in the corresponding types. Top DEG lists from both types (FIG. 1A) were selected and included as participating components in the network model. Further, the Cancer Cell Lines Encyclopedia (CCLE) and Breast Cancer International Consortium (METABRIC) used the DEG classification method to identify patterns of differentially expressed genes in the corresponding types of TCGA (FIGS. 1B to 1G).

As a result, as shown in FIG. 2A, a network model specific to and essential for basal-like breast cancer and luminal type A breast cancer, consisting of a total of 30 nodes and 73 links, was constructed through the above-described various data analyses.

Further, as shown in FIGS. 2B to 2E, in order to create a cell-specific network by substituting the network into an actual breast cancer cell line, functional genomic profiles of each cell line were constructed using gene copy number variation (CNA), which is genomic information of each cell line, mRNA expression amount, and gene mutation information in CCLE data.

Further, as shown in FIG. 2F, networks having specific genomic information of the cell line were constructed by substituting the result into the corresponding cell line.

### Example 2. Target genes for cell that induce BLT by network control method using LDOI

Based on the results of Example 1, the present inventors analyzed whether a basal-like network was reprogrammed and transdifferentiated into a luminal type A network when a certain gene node was controlled through a logical domain of influence (LDOI)-based target control strategy, one of the network control methods by ERa, a representative characteristic of luminal type A, and the activity of nodes highly expressed in luminal type A.

As a result, as shown in Table 1 below, it was confirmed that reprogramming to the luminal type A was possible when BCL11A BCL11A (B-cell lymphoma/leukemia 11A; NM_022893) and HDAC1/2 (Histone deacetylase 1/2; NM_004964/NM_001527) were suppressed as the most optimal combination.

**[Table 1]**

| Desired state | | |
|---|---|---|
| ON: ERa, C6orf97, KRT18, FOXA1,ESR1, CA12, ANXA9, GATA9 | | |
| OFF: EGFR, AKT, ERK1/2 | | |
| No. | LDOI Target solution | Total number of solution found |
| *Network without any genomic alteration* | | |
| 1 | BCL11A **OFF** & HDAC1/2 **OFF** | 46 |
| 2 | PI3K **OFF** & HDAC112 **OFF** | 11 |
| 3 | KRAS **OFF** & HDAC1/2 **OFF** | 10 |

| *BT20 network* | | |
|---|---|---|
| 1 | BCL11A **OFF** & HDAC1/2 **OFF** | 16 |
| 2 | KRAS **OFF** & HDAC1/2 **OFF** | 12 |

| *MDA-MB231 network* | | |
|---|---|---|
| 1 | BCL11A **OFF** & HDAC1/2 **OFF** | 32 |
| 2 | PI3K **OFF** & HDAC1/2 **OFF** | **4** |

### Example 3. Identification of mechanism of BLT induced by BCL11A KO and HD AC 1/2 KO.

Based on the results of Example 2, the present inventors confirmed the mechanism of BLT induced by knockouts of BCL11A and HDAC1/2.

The extended network provided by the LDOI-based targeted control strategy resembles a hypergraph integrating the regulatory interactions and dynamics of the network. Therefore, the interaction of BCL11A and HDAC1/2 with other network components was analyzed using the extended network of the BLT model. As a result, the expanded network includes the states of all network components and all possible initial states with the luminal A phenotype as the target state.

Next, as shown in FIG. 3A, after each perturbation, HD AC 1/2 KO or [~ HDAC1/2], BCL11A KO or [-BCL11A] and [~ BCL11A & ~ HDAC1/2] or both BCL11A and HDAC1/2 KO, transition was temporarily explored to analyze how BLT occurs.

Further, as shown in FIGS. 3B and 3C, it was confirmed that when both BCL11A and HDAC1/2 were inhibited, the luminal type A breast cancer-related node increased and the activity of the basal-like breast cancer-related node decreased.

### Example 4. Dynamic stability of modified attractors after BCL11A and HDAC1/2 KO

The present inventors compared the attractor landscape of the network constructed when controlling each target gene selected through the network analysis and the phenotype landscape.

The attractor landscape analysis was performed by mathematically analyzing the watershed of each attractor to investigate the dynamic stability of the state and the effect of the target on the BLT. An attractor is a state within a network model that can be defined as the binary activity of molecules in the network.

Further, the phenotype of each resultant attractor was defined, and the percentage of each basal-like type and luminal type A phenotype was calculated when the corresponding target genes were controlled in two different cell line-specific networks.

As a result, as shown in FIG. 4, it can be seen that the phenotype is changed to 100% of the luminal type A when all genes are controlled rather than when each gene is controlled.

### Example 5. Effects of BCL11A and HDAC1/2 KOs on expression of ESR1 mRNA and ERα

In order to confirm the effects of BCL11A and HDAC1/2 KOs on the expression of ESR1 mRNA and ERα, the present inventors confirmed changes in the expression levels of ESR1 mRNA and Erα when BCL11A and HDAC1/2 genes were inhibited in basal-like type and triple-negative breast cancer cell lines MDA-MB231 (ER-/PR-/HER2-) and BT20 (ER-/PR-/HER2-) cells. At this time, shBCL11A (GCATAGACGATGGCACTGTTA; SEQ ID NO: 1) designed to target the BCL11A gene was used as the BCL11A inhibitor, and romidepsin (R), a drug targeting HD AC 1/2, was used as the HDAC1/2 inhibitor.

Changes in the expression of ESR1 mRNA, ERα, and other EGFR-related proteins were observed after treatment with the inhibitors.

As a result, as shown in FIGS. 5A to 5D, it was confirmed that ESR1 mRNA expression increased in each cell line when each gene was inhibited, and the expression increased more than when all genes were inhibited.

Further, the protein expression level of ERa, a luminal type A marker receptor, was checked to confirm whether the result was the same as that of the simulation analysis.

As a result, as shown in FIGS. 5E and 5F, the ERa expression level increased when each gene was inhibited, and the protein amount was higher when all genes were inhibited.

That is, it can be seen that BCL11A and HDAC1/2 KO induce transdifferentiation of the basal-like and triple-negative cell lines into luminal type A cell lines to express ESR1 and ERα, which are characteristic of luminal type A.

Further, to confirm whether it is due to the EGFR-ERK1/2 cell signaling pathway, in each cell line, when the level of phosphorylation representing EGFR and ERK1/2 activity was measured, it was confirmed that the expression decreased.

### Example 6. Tamoxifen drug response after BCL11A and HDAC1/2 KOs

Based on the results of Example 5, in order to investigate that transdifferentiation of basal-like cell lines into luminal type A cell lines is induced by BCL11A and HDAC1/2 KOs, they can become sensitive to anti-hormonal therapy targeting ERα, the present inventors treated basal-like and triple-negative breast cancer cell lines, MDA-MB231 and BT20 cells, with tamoxifen, an ERα-targeting drug for patients with luminal type breast cancer, as an anti-hormonal agent.

Briefly, it is as follows: Cells were plated in 1 × 10⁴ cells (3 repetitions for each condition; triplicate), after 24 hours, the cells were treated with the tamoxifen drug (10 uM) and monitored by taking pictures at 3-hour intervals using Incucyte for 72 hours. After that, the confluency of the cells in the pictures was calculated and the sensitivity of the cells to various drugs was graphed.

As a result, as shown in FIGS. 6A to 6D, control cells (shScrambled) did not show sensitivity, and BCL11A KD (knockdown) cells using shBCL11A and HD AC 1/2 suppressor cells using romidepsin (HDAC1/2 inhibitor) showed some sensitivity to Tamoxifen (Tam).

On the other hand, cells treated with both shBCL11A and romidepsin showed the highest sensitivity to tamoxifen.

### Example 7. Tamoxifen drug response after BCL11A and HDAC1/2 overexpression

The present inventors confirmed the tamoxifen drug response in human breast cancer cell lines T47D and MCF7 upon overexpression (OE) of BCL11A and HDAC1/2.

As a result, as shown in FIGS. 7A to 7D, overexpression of BCL11A and/or HD AC 1/2 in the luminal A (ER+/HER2-) T47D cell line did not show a dramatic change in the expression level of ESR1 mRNA.

Meanwhile, increased ERα protein expression, decreased phosphorylated EGFR and phosphorylated ERK1/2 were observed after BCL11A or HDAC1/2 overexpression. When these genes were combined and overexpressed in T47D cells, their effect was synergistic.

Further, as shown in FIGS. 7E to 7G, the reduced expression level of ERα protein in luminal type A cells after overexpression of BCL11A and HDAC1/2 indicates that luminal type A cells were reprogrammed into basal-like cells that did not respond to tamoxifen.

Also, while control cells responded to tamoxifen, cells overexpressing BCL11A-OE, HDAC1/2-OE, or both did not show sensitivity to tamoxifen.

Further, as shown in FIGS. 7H to 7K, the same results were confirmed in another luminal type A (ER+/HER2-) MCF7 cell line.

These results indicate that both BCL11A and HDAC1/2 OEs may induce reverse-BLT through upregulated activities of ERK1/2 and EGFR and downregulated expression of ERα.

Accordingly, it can be seen that when the corresponding genes are overexpressed in the luminal type A cell line, basal-like properties can be obtained.

In conclusion, the target genes of the present invention are inhibited to induce BLT, thereby reprogramming the malignant breast cancer type into luminal type A that responds to ERα target drugs such as tamoxifen, and conventional drugs used for the treatment of luminal type A patients are used to guarantee an effective anti-hormone treatment effect in addition to anticancer chemotherapy.

### Example 8. Survival rates of patients according to BCL11A and HD AC 1/2 expression

The present inventors additionally analyzed the METABRIC and TCGA databases to confirm the effect of BCL11A and HDAC1/2 expression on patient survival rates.

As a result, as shown in FIGS. 8A to 8R, patients with BCL11A, HDAC1 or HDAC2 cancer cells overexpressed in the METABRIC and TCGA databases showed a significantly lower survival rate compared to patients with relatively low expression of BCL11A, HDAC1 or HDAC2 cancer cells.

That is, these increased ESR1 mRNA and ERα protein expression and drug response to tamoxifen suggest that depletion of BCL11A and HDAC1/2 may induce BLT in patients.

### Example 9. Patients' drug response to tamoxifen according to BCL11A and HD AC 1/2 expression

The present inventors additionally analyzed gene set variation analysis (GSVA) to confirm the effect of BCL11A and HDAC1/2 expression on tamoxifen drug response in breast cancer patients.

As a result, as shown in FIG. 9A, the patient group with low expression of the two genes showed higher sensitivity to the tamoxifen drug compared to the patient group with high BCL11A and HD AC 1/2 expression.

Further, as shown in FIGS. 9B and 9C, the gene expression patterns of the BCL11A and HDAC1/2 overexpressing patient groups were enriched in the EGFR signaling pathway or basal-like gene group, whereas the gene expression patterns of the BCL11A and HD AC 1/2 low expression patient groups were enriched in the ER signaling pathway or the luminal type A gene group.

Therefore, these results demonstrate that the expression levels of BCL11A and HD AC 1/2 as target genes were analyzed and controlled (inhibited) before treatment, for patients with ER-negative malignant breast cancer such as basal-like breast cancer or triple-negative breast cancer in clinical practice, thereby reprogramming into luminal type A patients capable of anti-ERa therapy by expressing estrogen receptor alpha (ERα), and then an effective anti-hormone treatment effect may be achieved by using a conventional treatment drug for luminal type A patients.

So far, certain parts of the present invention have been described in detail, and it is clear to those skilled in the art that these specific descriptions are only preferred embodiments, and the scope of the present invention is not limited thereby. Accordingly, the substantial scope of the present invention is defined by the appended claims and their equivalents.

## Claims

1. A composition for transdifferentiating estrogen receptor (ER)-negative breast cancer to luminal breast cancer, comprising B-cell lymphoma/leukemia 11A (BCL11A) inhibitor and Histone deacetylase 1/2 (HDAC1/2) inhibitor as active ingredients.

2. The composition of claim 1, wherein the ER-negative breast cancer is negative for at least one selected from the group consisting of progesterone receptor (PR) and human epidermal factor receptor 2 (HER2).

3. The composition of claim 1, wherein the ER-negative breast cancer is triple-negative breast cancer (TNBC) or basal-like breast cancer (BLBC).

4. The composition of claim 1, wherein the luminal breast cancer is at least one selected from the group consisting of luminal type A and luminal type B.

5. The composition of claim 1, wherein the BCL11A inhibitor includes at least one selected from the group consisting of an antisense oligonucleotide, small interference RNA (siRNA), short hairpin RNA (shRNA), microRNA (miRNA) and ribozyme which complementarily bind to an mRNA of BCL11A gene, and
wherein the HD AC 1/2 inhibitor includes at least one selected from the group consisting of an antisense oligonucleotide, small interference RNA (siRNA), short hairpin RNA (shRNA), microRNA (miRNA) and ribozyme which complementarily bind to an mRNA of HD AC 1/2 gene.

6. The composition of claim 1, wherein the BCL11A inhibitor includes at least one selected from the group consisting of a compound, peptide, peptidomimetic, substrate analog, aptamer, and antibody which specifically bind to BCL11A protein, and
wherein the HD AC 1/2 inhibitor includes at least one selected from the group consisting of a compound, peptide, peptidomimetic, substrate analog, aptamer, and antibody which specifically bind to HD AC 1/2 protein.

7. The composition of claim 1, wherein the BCL11A inhibitor and HDAC1/2 inhibitor reduce an expression level or activity of epidermal growth factor receptor (EGFR) and extracellular signal-regulated kinase 1/2 (ERK1/2); and increase an expression level or activity of estrogen receptor alpha (ERα).

8. A composition for enhancing sensitivity to anticancer agent for ER-negative breast cancer, comprising the composition according to any one of claims 1 to 7 as an active ingredient.

9. An anticancer adjuvant for ER-negative breast cancer, comprising the composition according to any one of claims 1 to 7 as an active ingredient.

10. A pharmaceutical composition for treating or preventing ER-negative breast cancer, comprising the composition according to any one of claims 1 to 7; and an anticancer agent as active ingredients.

11. The composition of claim 10, wherein the anticancer agent includes at least one selected from the group consisting of a selective estrogen receptor modulator (SERM), a selective estrogen receptor degrader (SERD) and an aromatase inhibitor (AI).

12. The composition of claim 11, wherein the anticancer agent is the SERM or the SERD, which is an ERα targeting agent.

13. A food composition for preventing or alleviating ER-negative breast cancer, comprising BCL11A inhibitor and HDAC1/2 inhibitor as active ingredients.

14. A method for inducing *in vitro* transdifferentiation of ER-negative breast cancer to luminal breast cancer, comprising treating a BCL11A inhibitor and an HD AC 1/2 inhibitor to an ER-negative breast cancer cell.

15. A method for screening a drug for enhancing sensitivity to anticancer agent for ER-negative breast cancer, comprising following steps:
(a) treating an isolated ER-negative breast cancer cell with a candidate material;
(b) measuring expression levels of BCL11A and HDAC1/2 in the ER-negative breast cancer cells treated with the candidate material; and
(c) determining that the candidate material can be used as a drug for enhancing sensitivity to anticancer agent for the ER-negative breast cancer when the expression levels of BCL11A and HDAC1/2 measured in step (b) are lower than those of the isolated malignant breast cancer cells that are not treated with the candidate material.

16. A method for transdifferentiating ER-negative breast cancer to luminal breast cancer, comprising administering the composition of any one of claims 1 to 7 to a subj ect.

17. A method for treating or preventing ER-negative breast cancer, comprising the composition of any one of claims 1 to 7 and an anticancer agent to a subject.
